# EUROPEAN PATENT APPLICATION

(11) **EP 4 342 883 A1**
(43) Date of publication of application: **27.03.2024**
(21) Application number: 22820275.0
(22) Date of filing: 08.06.2022
(51) Int. Cl.: C07D 209/80, C07D 209/86, C07D 239/02, C07D 251/02, C07D 251/24, C07D 307/91, C07D 307/92, C07D 333/54, C07D 333/74, C07D 333/76, C07D 403/04, C07D 403/10, C07D 403/14, C07D 405/04, C07D 405/10, C07D 407/04, C07D 407/10, C07D 409/04, C07D 409/10, C07D 409/12, C07D 409/14

(54) **PHOTOELECTRIC CONVERSION ELEMENT MATERIAL FOR IMAGING ELEMENT AND PHOTOELECTRIC CONVERSION ELEMENT FOR IMAGING ELEMENT**

(30) Priority: 11.06.2021 JP 2021098353; 07.01.2022 JP 2022001855
(71) Applicant: Tosoh Corporation, Yamaguchi 746-8501 (JP)
(72) Inventor: MORINAKA, Yuta, Ayase-Shi, Kanagawa 2521123 (JP); SHONO, Tomohiro, Shunan-shi, Yamaguchi 746-8501 (JP); TAKAHASHI, Yasuhiro, Ayase-Shi, Kanagawa 2521123 (JP); NOMURA, Keisuke, Tokyo 1058623 (JP); OKADA, Takeshi, Ayase-Shi, Kanagawa 2521123 (JP); ONO, Youhei, Ayase-Shi, Kanagawa 2521123 (JP); TANAKA, Tsuyoshi, Shunan-shi, Yamaguchi 746-8501 (JP)
(74) Representative: TBK
(86) International application number: PCT/JP2022/023166
(87) International publication number: WO 2022/260096

(57) **Abstract**

Provided is a photoelectric conversion element material that is for an imaging element and that contributes to production of a photoelectric conversion element that is for an imaging element and that exhibits excellent dark current and external quantum efficiency. The present invention uses a photoelectric conversion element material that is for an imaging element and that contains a compound including a backbone that has a cove region in the molecular structure thereof and that optionally includes a substitution. However, examples of the compound does not include unsubstituted dibenzo[g,p]chrysene. Preferable as the abovementioned material is a photoelectric conversion element material that is for an imaging element and that has a specific structure represented by formula (1).

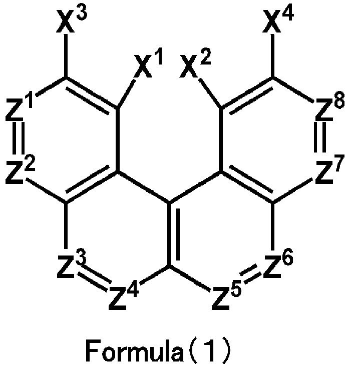

## Description

### TECHNICAL FIELD

The present invention relates to a photoelectric conversion element material for imaging elements, and a photoelectric conversion element for imaging elements.

### BACKGROUND ART

Photoelectric conversion elements for imaging elements are used in applications such as mobile telephones and cameras, and the development thereof is being vigorously conducted.

The demand from the market for photoelectric conversion elements for imaging elements in recent years is increasing more and more, and materials superior in all of dark current, external quantum efficiency and response speed has been demanded. Under this situation, the exploration and consideration has been continuing in the possibility of various polycyclic compounds as the core of novel materials. As polycyclic compounds, Patent Document 1 discloses derivatives with benzothienobenzothiophene as the core. In addition, Patent Document 2 discloses various cores in addition to benzothienobenzothiophene. Patent Document 3 discloses unsubstituted dibenzo[g,p] chrysene.

### Citation List

### Patent Document

Patent Document 1: PCT International Publication No. WO2015/163349
Patent Document 2: PCT International Publication No. WO2020/022421
Patent Document 3: Japanese Unexamined Patent Application, Publication No. 2010-258438

### DISCLOSURE OF THE INVENTION

### Problems to be Solved by the Invention

The object of an aspect of the present invention is to provide a photoelectric conversion element material for imaging elements and a photoelectric conversion element for imaging elements made using a compound having this novel core, while exploration and consideration is continuing for the possibility of various polycyclic compounds as the core of a novel material.

Another object of an aspect of the present invention is to provide a photoelectric conversion element material for imaging elements, a photoelectric conversion element transport material for imaging elements, and a photoelectric conversion element hole transport material for imaging elements contributing to the production of a photoelectric conversion element for imaging elements having low dark current and high external quantum efficiency. In addition, another object of an aspect of the present invention is to provide a photoelectric conversion element for imaging elements having low dark current and high external quantum efficiency. Incidentally, Patent Document 3 has a disclosure of using unsubstituted dibenzo[g,p]chrysene as a crystalline layer between the photoelectric conversion layer and upper electrode. However, with Patent Document 3, there is no mention of the molecular structure characteristics of dibenzo[g,p]chrysene or the amorphous filmi containing dibenzo[g,p]chrysene. Additionally, the dibenzo[g,p]chrysene disclosed in Patent Document 3 in no way provides knowledge of improving the performance of a photoelectric conversion element for imaging elements.

### Means for Solving the Problems

According to an aspect of the present invention, a photoelectric conversion element material for imaging elements is provided which includes a compound containing a backbone having a cove region in the molecular structure and in which this backbone may be substituted, provided that dibenzo[g,p]chrysene is not included as the compound.

According to another aspect of the present invention, a photoelectric conversion element for imaging elements in which the backbone is represented by Formula (1) below is provided.

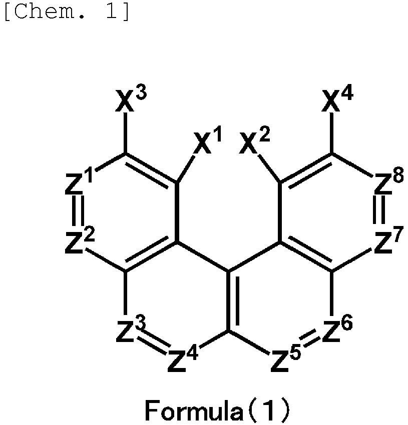

In Formula (1),
X¹ to X⁴ each independently represent a hydrogen atom or a substituent;
X¹ to X⁴ do not bond together to form a ring;
a plurality of X¹ to X⁴ may be the same or different;
Z¹ to Z⁸ each independently represent a nitrogen atom or a carbon atom that may have a substituent;
at least six of Z¹ to Z⁸ are carbon atoms that may have a substituent;
a plurality of Z¹ to Z⁸ may be the same or different; and
any of Z¹ to Z⁸, in a case of being a carbon atom having a substituent, may bond with a substituent on a carbon atom of any one or two of Z¹ to Z⁸ adjacent to the carbon atom to form a ring.

According to another aspect of the present invention, a photoelectric conversion element material for imaging elements according to the above aspect is provided which is a photoelectric conversion element transport material for imaging elements, or a photoelectric conversion element charge blocking material for imaging elements.

According to another aspect of the present invention, a photoelectric conversion element material for imaging elements according to the above aspect is provided which is a photoelectric conversion element hole transport material for imaging elements or a photoelectric conversion element electron blocking material for imaging elements.

According to another aspect of the present invention, a photoelectric conversion element for imaging elements is provided which contains the photoelectric conversion element material for imaging elements according to the above aspect.

### Effects of the Invention

According to an aspect of the present invention, it is possible to provide a photoelectric conversion element material for imaging elements, a photoelectric conversion element hole transport material for imaging elements and a photoelectric conversion element electron blocking material for imaging elements contributing to the production of a photoelectric conversion element for imaging elements that is superior in dark current and external quantum efficiency.

According to another aspect of the present invention, it is possible to provide a photoelectric conversion element for imaging elements that is superior in dark current and external quantum efficiency.

### BRIEF DESCRIPTION OF THE DRAWINGS

Fig. 1 is a schematic cross-sectional diagram showing an example of a laminate configuration of the photoelectric conversion element for imaging elements containing the photoelectric conversion element material for imaging elements according to an aspect of the present invention;
Fig. 2 is a schematic cross-sectional diagram showing a laminate configuration of an Element Example A-1; and
Fig. 3 is a schematic cross-sectional diagram showing a lamination configuration of an Element Example B-1.

### PREFERRED MODE FOR CARRYING OUT THE INVENTION

Hereinafter, a photoelectric conversion element material for imaging elements related to an aspect of the present invention will be explained in detail.

### <Photoelectric conversion element material for imaging elements>

An aspect of the present invention is a photoelectric conversion element material for imaging elements including a compound containing a backbone having a cove region in the molecular structure, in which this backbone may be substituted, provided that unsubstituted dibenzo[g,p]chrysene is not included as this compound. Cove region is a name indicating a specific molecular structure region in a polycyclic compound, and indicates the region disclosed in Non-patent Document (Chemical Science, 2019, 10, 4025). It should be noted that compounds are also encompassed by "material" in the present disclosure.

Cove region refers to a region of a [4] helicene backbone having 4 benzene ring condensed, which is cut in a bay shape formed by four sides combining one side of each benzene ring. Photoelectric conversion element material for imaging elements may have at least one of hydrogen atoms and carbon atoms in the above [4] helicene backbone having a cove region substituted. In addition, in the [4] helicene backbone, a heterohelicene backbone in which part of the benzene rings are substituted with a heterocycle is also included. In the case of the photoelectric conversion element material for imaging elements including a heterohelicene backbone having a cove region, at least one among hydrogen atoms, carbon atoms, oxygen atoms, sulfur atoms and nitrogen atoms in the heterohelicene backbone may be substituted.

In the embodiment in which the backbone having the cove region is substituted, the substituent possessed by the backbone is preferably a charge transport substituent. The details of the charge transport substituent are described later, and the specific substituents described later can be used as is in the present embodiment.

It should be noted that it can also be considered that a cove region is partially included in a [5] helicene backbone in which five benzene rings are condensed, or a [6] helicene backbone in which six benzene rings are condensed; however, these are regions called fjord regions, and do not correspond to a cove region.

Cove region in the present invention is a region possessed by the compound represented by Formula (1), and more specifically, indicates the region indicated by the bold line in Formula (1'). In other words, an aspect of the present invention is preferably a photoelectric conversion element material for imaging elements including a compound having the backbone shown by Formula (1) in which this backbone may be substituted, provided that unsubstituted dibenzo[g,p]chrysene is not included as this compound.

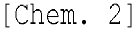

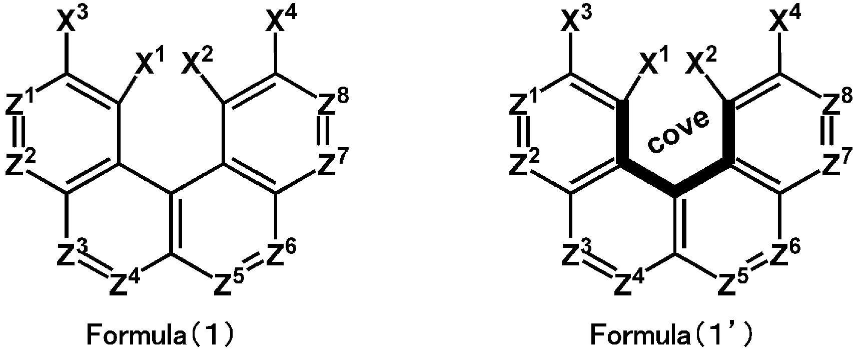

In Formula (1),
X¹ to X⁴ each individually represent a hydrogen atom or substituted group;
X¹ to X⁴ do not form a ring by bonding to each other;
a plurality of X¹ to X⁴ may be the same or different;
Z¹ to Z⁸ each individually represent a nitrogen atom, or a carbon atom which may have a substituent;
among Z¹ to Z⁸, at least six are carbon atoms which may have a substituent;
a plurality of Z¹ to Z⁸ may be the same or different; and
any of Z¹ to Z⁸, in the case of being a carbon atom which may have a substituent, may bond with a substituent on a carbon atom of any one or two Z¹ to Z⁸ adjacent to this carbon atom, and form a ring.

The definition of the above Formula (1), and preferred specific examples thereof are each as follows.

### <Regarding X¹ to X⁴>

X¹ to X⁴ each independently represent a hydrogen atom or substituent. X¹ to X⁴ do not form a ring with each other, and have the cove region in the molecular structure by not making a ring formation. From the viewpoint of producing a photoelectric conversion element material for imaging elements, X¹ and X² are both preferably a hydrogen atom.

### <Regarding Z¹ to Z⁸>

Z¹ to Z⁸ each independently represent a nitrogen atom or a carbon atom which may have a substituent. Among Z¹ to Z⁸, at least six are preferably a carbon atom that may have a substituent, at least seven are more preferably a carbon atom that may have a substituent, and all are particularly preferably a carbon atom that may have a substituent. Additionally, Z³ and Z⁴ are carbon atoms that may have a substituent, and the substituents of Z³ and Z⁴ preferably bond with each other to form a ring.

The photoelectric conversion element material for imaging elements which is an aspect of the present invention more specifically preferably includes a compound represented by any of the following Formulas (2A) to (5B).

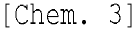

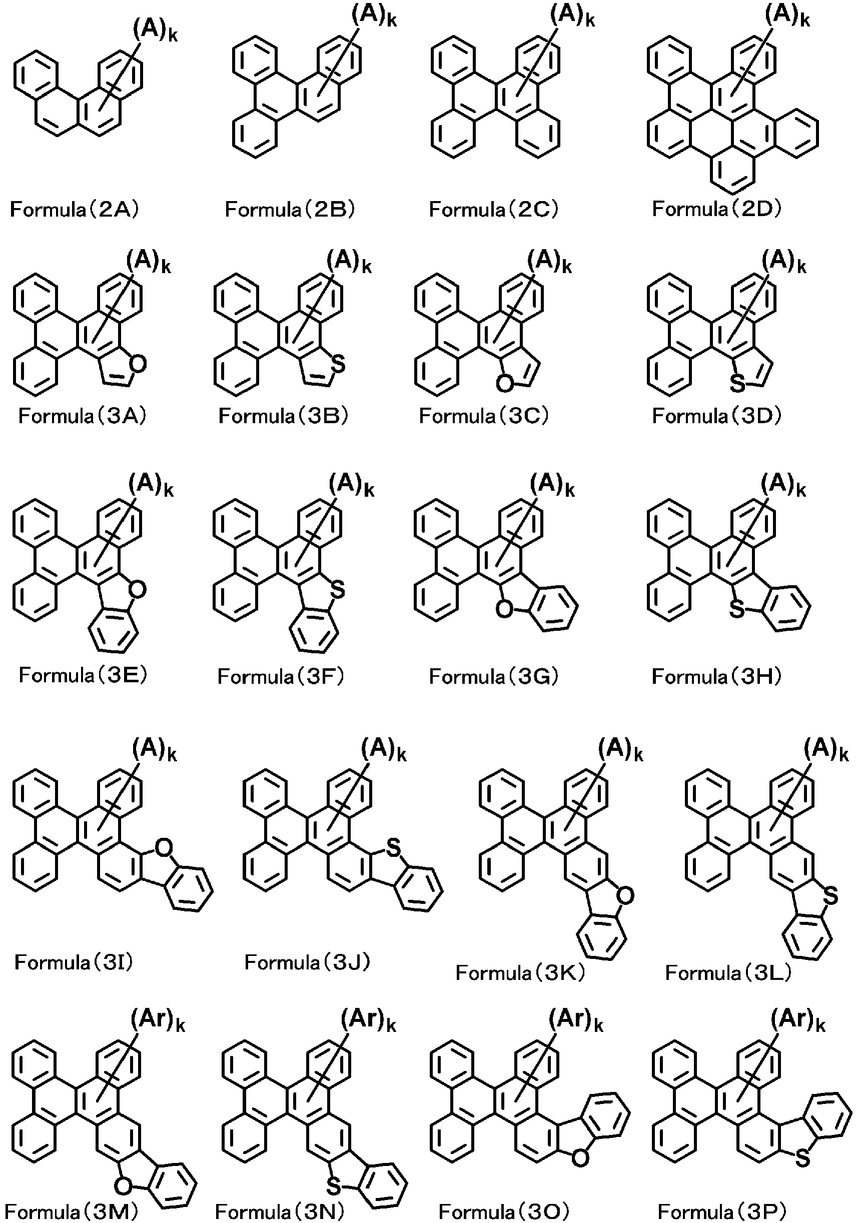

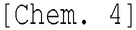

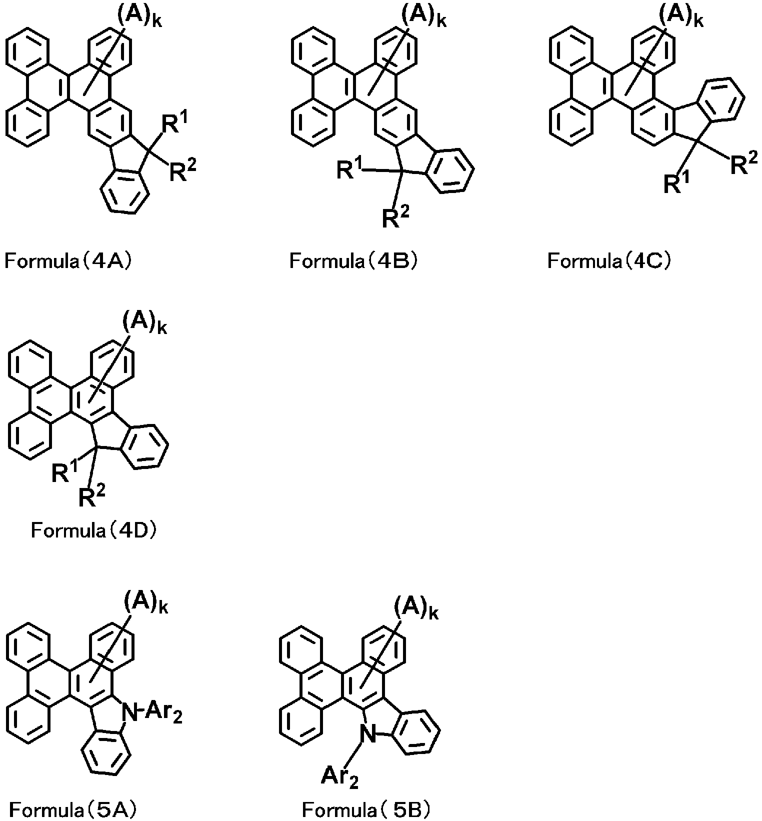

In Formulas (2A) to (5B),
A represents a charge transport substituent;
Ar₂ represents a monocyclic, linked, or condensed aromatic hydrocarbon group of 6 to 30 carbon atoms that may have a substituent, a monocyclic, linked or condensed heteroaromatic group of 3 to 36 carbon atoms that may have a substituent, and a linear or branched alkyl group of 1 to 18 carbon atoms;
R¹ and R² are each independently
a hydrogen atom;
a deuterium atom;
a monocyclic, linked or condensed aromatic hydrocarbon group of 6 to 30 carbon atoms that may have a substituent;
a monocyclic, linked or condensed heteroaromatic group of 3 to 36 carbon atoms that may have a substituent;
a linear or branched alkyl group of 1 to 18 carbon atoms; or
a linear or branched alkoxy group of 1 to 18 carbon atoms;
R¹ and R² may bond with each other to form a ring;
K represents an integer of 1 to 4; and
a plurality of A may be the same or different.

The definition of the charge transport substituent in the photoelectric conversion element material for imaging elements (2A) to (5B), and preferred specific examples thereof are each as follows.

### <Regarding Charge Transport Substituent>

Charge transport substituent is a substituent having a function of transporting charge. Charge is holes, electrons or both.

As the charge transport substituent, for example, the substituents shown by (a-1) to (a-16) below can be exemplified.
(a-1) deuterium atom,
(a-2) fluorine atom, chlorine atom, bromine atom, iodine atom,
(a-3) trifluoromethyl group,
(a-4) pentafluoroethyl group,
(a-5) cyano group,
(a-6) nitro group,
(a-7) hydroxy group,
(a-8) thiol group,
(a-9) monocyclic, linked or condensed aromatic hydrocarbon group of 6 to 30 carbon atoms that may have a substituent,
(a-10) monocyclic, linked or condensed heteroaromatic group of 3 to 36 carbon atoms that may have a substituent,
(a-11) phosphine oxide group that may have a substituent,
(a-12) silyl group that may have a substituent,
(a-13) boronyl group that may have a saturated hydrocarbon group of 2 to 10 carbon atoms,
(a-14) a linear or branched alkyl group of 1 to 18 carbon atoms,
(a-15) a linear or branched alkoxy group of 1 to 18 carbon atoms,
(a-16) trifluoromethylsulfonyloxy group, or
(a-17) group represented by Formula (6) or (6') below.

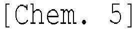

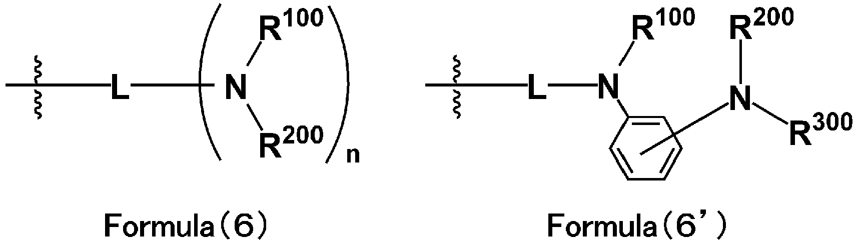

In the formula,
R¹⁰⁰ to R³⁰⁰ are each independently
(r-1) hydrogen atom, (R-2) deuterium atom,
(r-3) monocyclic, linked, or condensed aromatic hydrocarbon group of 6 to 30 carbon atoms that may have a substituent,
(r-4) monocyclic, linked or condensed heteroaromatic group of 3 to 36 carbon atoms that may have a substituent, or
(r-5) a linear or branched alkyl group of 1 to 18 carbon atoms,
L each independently represent
a phenylene group that may be substituted by a methyl group or phenyl group,
a naphthylene group that may be substituted by a methyl group or phenyl group,
a biphenylene group that may be substituted by a methyl group or phenyl group, or
a single bond;
n represents 1 or 2,
in the case of L being a single bond, n is 1,
in the case of L not being a single bond, n is 1 or 2; and
in the case of n being 2, a plurality of R¹⁰⁰ to R²⁰⁰ may be the same or different.

### <Regarding (a-9)>

As the monocyclic, linked or condensed aromatic hydrocarbon group of 6 to 30 carbon atoms which is (a-9), for example, a phenyl group, biphenylyl group, terphenylyl group, naphthyl group, fluorenyl group, anthryl group, phenanthryl group, benzofluorenyl group, triphenylenyl group, spirobifluorenyl group, diphenylfluorenyl group, and dibenzo[g,p]chrysenyl group can be exemplified. In addition, the monocyclic, linked or condensed aromatic hydrocarbon group of 6 to 30 carbon atoms is preferably a monocyclic, linked or condensed aromatic hydrocarbon group of 6 to 18 carbon atoms.

It should be noted that, in the case of the aromatic hydrocarbon group of (a-9) having a substituent, this substituent is each independently preferably a fluorine atom, chlorine atom, bromine atom, iodine atom, cyano group, nitro group, hydroxy group, thiol group, a phosphine oxide group which may have a substituent, a silyl group which may have a substituent, a boronyl group which may have a saturated hydrocarbon group of 2 to 10 carbon atoms, a linear or branched alkyl group of 1 to 18 carbon atoms, a linear or branched alkoxyl group of 1 to 18 carbon atoms, or a trifluoromethylsulfonyloxy group.

As the phosphine oxide group, an unsubstituted phosphine oxide group or a phosphine oxide group having a substituent can be exemplified. It is preferably a phosphine oxide group having a substituent.

As the phosphine oxide group having a substituent, it is preferably a monocyclic, linked or condensed aromatic hydrocarbon group of 6 to 18 carbon atoms, or a phosphine oxide group having a condensed heteroaromatic group. More specifically, for example, a group substituted by two aryl groups such as diphenyl phosphine oxide can be exemplified.

As the silyl group, an unsubstituted silyl group or a silyl group having a substituent can be exemplified. It is preferably a silyl group having a substituent.

As the silyl group having a substituent, it is preferably a silyl group having a monocyclic, linked or condensed aromatic hydrocarbon group of 6 to 18 carbon atoms, or a condensed heteroaromatic group. More specifically, for example, a group substituted by three aryl groups such as triphenyl silyl group can be exemplified.

As the boronyl group that may have a saturated hydrocarbon group of 2 to 10 carbon atoms, for example, a dihydroxyboryl group (-B(OH)₂), 4,4,5,5-tetramethyl-[1,3,2]-dioxaborolanyl group, 5,5-dimethyl-[1,3,2]-dioxaborinane group and the like can be exemplified.

As the linear or branched alkyl group of 1 to 18 carbon atoms, for example, a methyl group, ethyl group, n-propyl group, i-propyl group, n-butyl group, sec-butyl group, tert-butyl group, pentyl group, n-hexyl group, cyclohexyl group, octyl group, decyl group, dodecyl group, octadecyl group and the like can be exemplified.

As the linear or branched alkoxy group of 1 to 18 carbon atoms, for example, a methoxy group, ethoxy group, n-propoxy group, i-propoxy group, n-butoxy group, sec-butoxy group, tert-butoxy group, pentyloxy group, n-hexyloxy group, cyclohexyloxy group, octyloxy group, decyloxy group, dodecyloxy group, octadecyloxy group and the like can be exemplified.

### <Regarding (a-10)>

As the monocyclic, linked or condensed heteroaromatic group of 3 to 36 carbon atoms which is (a-10), it is a monocyclic, linked or condensed heteroaromatic group of 3 to 36 carbon atoms containing at least one atom selected from the group consisting of an oxygen atom, nitrogen atom and sulfur atom on the aromatic ring.

As the heteroaromatic group, for example, a pyrrolyl group, thienyl group, furyl group, imidazolyl group, pyrazolyl group, thiazolyl group, isothiazolyl group, oxazolyl group, isoxazolyl group, pyridyl group, phenylpyridyl group, pyridylphenyl group, pyrimidyl group, pyrazyl group, 1,3,5-triazyl group, 1,3,5-triacylphenyl group, 1,3,5-triazilbiphenyl group, 4,6-diphenyl-1,3,5-triazyl group, indolyl group, benzothienyl group, benzofuranyl group, benzimidazolyl group, indazolyl group, benzothiazolyl group, benzisothiazolyl group, 2,1,3-benzothiadiazolyl group, benzoxazolyl group, benzisoxazolyl group, 2,1,3-benzoxadiazolyl group, quinolyl group, isoquinolyl group, quinoxalyl group, quinazolyl group, carbazolyl group, 9-phenylcarbazolyl group, 9-(4-biphenylyl)carbazolyl group, dibenzothienyl group, dibenzofuranyl group, phenoxazinyl group, phenothiazinyl group, phenazine group, thianthrenyl group and the like can be exemplified.

It should be noted that, in the case of the heteroaromatic group of (a-10) having a substituent, this substituent is preferably a cyano group, fluorine atom, trifluoromethyl group, linear or branched alkyl group of 1 to 18 carbon atoms, linear or branched alkoxy group of 1 to 18 carbon atoms, or trifluoromethylsulfonyloxy group.

As the linear or branched alkyl group of 1 to 18 carbon atoms, the same as the linear or branched alkyl group of 1 to 18 carbon atoms exemplified in the aforementioned (a-9) can be exemplified. As the linear or branched alkoxy group of 1 to 18 carbon atoms, the same as the linear or branched alkoxy group of 1 to 18 carbon atoms exemplified in the aforementioned (a-9) can be exemplified.

### <Regarding (a-11)>

As the phosphine oxide group which is (a-11), an unsubstituted phosphine oxide group or a phosphine oxide group having a substituent can be exemplified. It is preferably a phosphine oxide group having a substituent. As the phosphine oxide group having a substituent, for example, the same as the phosphine oxide group exemplified in the aforementioned (a-9) can be exemplified.

### <Regarding (a-12)>

As the silyl group which is (a-12), an unsubstituted silyl group or a silyl group having a substituent can be exemplified. It is preferably a silyl group having a substituent. As the silyl group having a substituent, for example, the same as the silyl group exemplified in the aforementioned (a-9) can be exemplified.

### <Regarding (a-13)>

As the boronyl group that may have a saturated hydrocarbon group of 2 to 10 carbon atoms which is (a-13), for example, the same as the boronyl groups exemplified in the aforementioned (a-9) can be exemplified.

### <Regarding (a-14)>

As the linear alkyl group of 1 to 18 carbon atoms which is (a-14), for example, the same as the linear or branched alkyl group of 1 to 18 carbon atoms exemplified in the aforementioned (a-9) can be exemplified.

### <Regarding (a-15)>

As the linear or branched alkoxy group of 1 to 18 carbon atoms which is (a-15), for example, the same as the linear or branched alkoxy group of 1 to 18 carbon atoms exemplified in the aforementioned (a-9) can be exemplified.

### <Regarding (a-17)>

(a-17) is a group represented by Formula (6) and (6'), and as mentioned above, the charge transport group may be a group represented by the above Formula (6) or (6'). In Formula (6) and (6'), the definitions of L, R¹⁰⁰ to R³⁰⁰, and n are as follows.

### <Regarding Formulas (6) and (6')>

In Formulas (6) and (6'), R¹⁰⁰ to R³⁰⁰ each independently represent a (r-1) hydrogen atom, (r-2) deuterium atom, (r-3) a monocyclic, linked or condensed aromatic hydrocarbon group of 6 to 30 carbon atoms which may have a substituent, (r-4) a monocyclic, linked or condensed heteroaromatic group of 3 to 36 carbon atoms which may have a substituent, or (r-5) a linear or branched alkyl group of 1 to 18 carbon atoms. In the case of R¹⁰⁰ to R³⁰⁰ having a substituent, R¹⁰⁰ to R³⁰⁰ may be substituted by one substituent, or may be substituted by two or more substituents.

The definition of the monocyclic, linked or condensed aromatic hydrocarbon group of 6 to 30 carbon atoms which is the above (r-3) is the same as the definition of the monocyclic, linked or condensed aromatic hydrocarbon group of 6 to 30 carbon atoms described in the above (a-9) except for the definition of the substituents thereof.

It should be noted that, in the case of the aromatic hydrocarbon group of (r-3) having a substituent, this substituent is preferably a deuterium atom, fluorine atom, linear or branched alkyl group of 1 to 18 carbon atoms, linear or branched alkoxy group of 1 to 18 carbon atoms, 9-carbazolyl group, dibenzothienyl group, dibenzofuranyl group, N,N-diphenylamino group or N,N-bis(4-biphenylyl)-amino group.

It should be noted that, as the above-mentioned linear or branched alkyl group of 1 to 18 carbon atoms, the same as the linear or branched alkyl group of 1 to 18 carbon atoms exemplified in the aforementioned (a-9) can be exemplified. As the branched linear or branched alkoxy group of 1 to 18 carbon atoms, the same as the linear or branched alkoxy group of 1 to 18 carbon atoms exemplified in the aforementioned (a-9) can be exemplified.

The definition of the monocyclic, linked or condensed heteroaromatic group of 3 to 36 carbon atoms which is the above (r-4) can be exemplified by the same as the monocyclic, linked or condensed heteroaromatic group of 3 to 36 carbon atoms exemplified in the aforementioned (a-10), except for the definition of substituents thereof. In addition, it is preferably a monocyclic, linked or condensed heteroaromatic group of 3 to 20 carbon atoms.

It should be noted that, in the case of the heteroaromatic group of (r-4) having a substituent, this substituent is preferably a deuterium atom, fluorine atom, linear or branched alkyl group of 1 to 18 carbon atoms, linear or branched alkoxy group of 1 to 18 carbon atoms, 9-carbazolyl group, dibenzothienyl group, dibenzofuranyl group, N,N-diphenylamino group or N,N-bis(4-biphenylyl)-amino group. These substituents, for example, are the same definition as the substituents of the aforementioned (r-3).

The definition of the linear or branched alkyl group of 1 to 18 carbon atoms which is the above (r-5) is the same as the definition shown in the above (a-9).

In Formulas (6) and (6'), L represents a phenylene group that may be substituted by a methyl group or phenyl group; a naphthylene group that may be substituted by a methyl group or phenyl group; a biphenylene group that may be substituted by a methyl group or phenyl group; or a single bond.

As the above phenylene group, for example, 1,2-phenylene group, 1,3-phenylene group, 1,4-phenylene group and the like can be exemplified.

As the above naphthylene group, for example, a naphthalene-1,2-diyl group, naphthalene-1,4-diyl group, naphthalene-1,8-diyl group, naphthalene-2,3-diyl group and the like can be exemplified.

As the biphenylene group, for example, biphenyl-4,4'-diyl group, biphenyl-4,3'-diyl group, biphenyl-4,2'-diyl group, bihenyl-3,3'-diyl group, biphenyl-3,2'-diyl group, biphenyl-2,2'-diyl group and the like can be exemplified.

In Formula (6) and (6'), n represents an integer of 1 or 2. In the case of L being a single bond, n is an integer of 1. In the case of L not being a single bond, n is an integer of 1 or 2. It should be noted that, in the case of n being 2, two of each of R¹⁰⁰ and R²⁰⁰ are present; however, they may be the same as each other, or may be different.

### <Regarding R¹ and R²>

In the case of R¹ and R² being (b-3) an aromatic hydrocarbon group having a substituent, or (b-4) a heteroaromatic group having a substituent, these substituents are preferably each independently a deuterium atom, fluorine atom, linear or branched alkyl group of 1 to 18 carbon atoms, or linear or branched alkoxy group of 1 to 18 carbon atoms. As specific examples of the linear or branched alkyl group of 1 to 18 carbon atoms, and linear or branched alkoxy group of 1 to 18 carbon atoms, although not particularly limited, the same as those exemplified in the aforementioned (a-9) can be exemplified respectively.

R¹ and R² may bond together to form a ring. For example, in the case of R¹ and R² being phenyl, it is possible to bond together to form a fluorene ring.

### (b-3): monocyclic, linked or condensed heteroaromatic group of 6 to 30 carbon atoms that may have a substituent

In R¹ and R² in Formulas (4A) to (4C), as the monocyclic, linked or condensed heteroaromatic group of 6 to 30 carbon atoms that may have a substituent, although not particularly limited, for example, a phenyl group, biphenyl group and the like can be exemplified.

### (b-4): monocyclic, linked or condensed heteroaromatic group of 3 to 36 carbon atoms that may have a substituent

In R¹ and R² in Formulas (4A) to (4C), as the monocyclic, linked or condensed heteroaromatic group of 3 to 36 carbon atoms, although not particularly limited, for example, a pyridyl group and the like can be exemplified.

### (b-5): linear or branched alkyl group of 1 to 18 carbon atoms

In Formulas (4A) to (4C), as the linear or branched alkyl group of 1 to 18 carbon atoms, the same as the linear or branched alkyl group of 1 to 18 carbon atoms exemplified in the above (a-9) can be exemplified.

### (b-6): linear or branched alkoxy group of 1 to 18 carbon atoms

In Formulas (4A) to (4C), as the linear or branched alkoxy group of 1 to 18 carbon atoms, the same as the linear or branched alkoxy group of 1 to 18 carbon atoms exemplified in the above (a-9) can be exemplified.

In the condensed ring compound represented by Formulas (4A) to (4C), in the point of ease of material obtaining, R¹ and R² are preferably each independently
a phenyl group, biphenyl group, pyridyl group, pyrimidyl group, or a group in which these groups are substituted by a methyl group or methoxy group; or
a methyl group, n-butyl group or n-hexyl group.

In addition, R¹ and R² more preferably are each independently
a phenyl group or a group in which the phenyl group is substituted by a methyl group or methoxy group; or
a methyl group.

### <Regarding k>

k is each independently an integer of 0 to 4. It should be noted that, in the case of k being 2 or more, a plurality of Ar are present; however, the plurality of Ar may be the same as each other, or may be different.

k is preferably 3 or less, and more preferably 2 or less. When k is no more than 3, the molecular weight is smaller compared to a compound in which k is 4 or more. As a result thereof, it is preferable because the sublimation temperature of the compound becomes lower, and the heat resistance stability during sublimation improves.

As specific examples of A, the groups of (1) to (24) shown below, etc. are given as preferred examples.
(1): methyl group, ethyl group, fluorine atom, chlorine atom, bromine atom, iodine atom, cyano group, nitro group, hydroxy group, thiol group, deuterium atom, methoxy group or trifluoromethylsulfonyloxy group
(2): phenyl group, 4-methylphenyl group, 3-methylphenyl group, 2-methylphenyl group, 2,4-dimethylphenyl group, 2,5-dimethylphenyl group, 3,4-dimethylphenyl group, 3,5-dimethyl phenyl group, 2,6-dimethylphenyl group, 2,3,5-trimethylphenyl group, 2,3,6-trimethylphenyl group, 2,4,6-trimethylphenyl group, 3,4,5-trimethylphenyl group, 4-hydroxyphenyl group, 3-hydroxy group, 2-hydroxyphenyl group, 3,5-dihydroxyphenyl group, 3,4-dihydroxyphenyl group, 4-methoxyphenyl group, 3-methoxyphenyl group, 2-methoxyphenyl group, 3,5-dimethoxyphenyl group, 3,4-dimethoxyphenyl group, 4-trifluoromethylsulfonyloxyphenyl group, 3-trifluoromethylsulfonyloxyphenyl group, 2-trifluoromethylsulfonyloxyphenyl group, 3,5-bis(trifluoromethylsulfonyloxy)phenyl group, 3,4-bis (trifluoromethylsulfonyloxy)phenyl group
(3): 4-biphenyl group, 3-biphenyl group, 2-biphenyl group, 2-methyl-1,1'-biphenyl-4-yl group, 3-methyl-1,1'-biphenyl-4-yl group, 2'- Methyl-1,1'-biphenyl-4-yl group, 3'-methyl-1,1'-biphenyl-4-yl group, 4'-methyl-1,1'-biphenyl-4-yl group, 2, 6-dimethyl-1,1'-biphenyl-4-yl group, 2,2'-dimethyl-1,1'-biphenyl-4-yl group, 2,3'-dimethyl-1,1'-biphenyl-4 -yl group, 2,4'-dimethyl-1,1'-biphenyl-4-yl group, 3,2'-dimethyl-1,1'-biphenyl-4-yl group, 2',3'-dimethyl- 1,1'-biphenyl-4-yl group, 2',4'-dimethyl-1,1'-biphenyl-4-yl group, 2',5'-dimethyl-1,1'-biphenyl-4-yl group, 2',6'-dimethyl-1,1'-biphenyl-4-yl group, 4-phenylbiphenyl group, 2-phenylbiphenyl group
(4): 1-naphthyl group, 2-naphthyl group, 2-methylnaphthalen-1-yl group, 4-methylnaphthalen-1-yl group, 6-methylnaphthalen-2-yl group, 4-(1-naphthyl)phenyl group, 4-(2-naphthyl)phenyl group, 3-(1-naphthyl)phenyl group, 3-(2-naphthyl)phenyl group, 3-methyl-4-(1-naphthyl)phenyl group, 3-methyl-4- (2-naphthyl)phenyl group, 4-(2-methylnaphthalen-1-yl)phenyl group, 3-(2-methylnaphthalen-1-yl)phenyl group, 4-phenylnaphthalen-1-yl group, 4-(2-methylphenyl)naphthalen-1-yl group, 4-(3-methylphenyl)naphthalen-1-yl group, 4-(4-methylphenyl)naphthalen-1-yl group, 6-phenylnaphthalen-2-yl group yl group, 4-(2-methylphenyl)naphthalen-2-yl group, 4-(3-methylphenyl)naphthalen-2-yl group, 4-(4-methylphenyl)naphthalen-2-yl group
(5): 2-fluorenyl group, 9,9-dimethyl-2-fluorenyl group, 9, 9'-spirobifluorenyl group, 9-phenanthryl group, 2-phenanthryl group, 11,11'-dimethylbenzo[a]fluorene-9-yl group, 11,11'-dimethylbenzo[a]fluoren-3-yl group, 11,11'-dimethylbenzo[b]fluoren-9-yl group, 11,11'-dimethylbenzo[b]fluorene-3-yl group, 11,11'-dimethylbenzo[c]fluoren-9-yl group, 11,11'-dimethylbenzo[c]fluoren-2-yl group, 3-fluoranthenyl group, 8-fluorenyl group oranthenyl group
(6): 1-imidazolyl group, 2-phenyl-1-imidazolyl group, 2-phenyl-3,4-dimethyl-1-imidazolyl group, 2,3,4-triphenyl-1-imidazolyl group, 2-(2-naphthyl)-3,4-dimethyl-1-imidazolyl group, 2-(2-naphthyl)-3,4-diphenyl-1-imidazolyl group, 1-methyl-2-imidazolyl group, 1-ethyl-2-imidazolyl group, 1-phenyl-2-imidazolyl group, 1-methyl-4-phenyl-2-imidazolyl group, 1-methyl-4,5-dimethyl-2-imidazolyl group, 1-methyl-4,5-diphenyl-2-imidazolyl group, 1-phenyl-4,5-dimethyl-2-imidazolyl group, 1-phenyl-4,5-diphenyl-2-imidazolyl group, 1-phenyl-4,5-divhenyl-2-imidazolyl group
(7): 1-methyl-3-pyrazolyl group, 1-phenyl-3-pyrazolyl group, 1-methyl-4-pyrazolyl group, 1-phenyl-4-pyrazolyl group, 1-methyl-5-pyrazolyl group, 1-phenyl-5-pyrazolyl group
(8): 2-thiazolyl group, 4-thiazolyl group, 5-thiazolyl group, 3-isothiazolyl group, 4-isothiazolyl group, 5-isothiazolyl group
(9): 2-oxazolyl group, 4-oxazolyl group, 5-oxazolyl group, 3-isoxazolyl group, 4-isoxazolyl group, 5-isoxazolyl group
(10): 2-pyridyl group, 3-methyl-2-pyridyl group, 4-methyl-2-pyridyl group, 5-methyl-2-pyridyl group, 6-methyl-2-pyridyl group, 3-pyridyl group, 4-methyl- 3-pyridyl group, 4-pyridyl group, 2-pyrimidyl group, 2,2'-bipyridin-3-yl group, 2,2'-bipyridin-4-yl group, 2,2'-bipyridin-5-yl group , 2,3'-bipyridin-3-yl group, 2,3'-bipyridin-4-yl group, 2,3'-bipyridin-5-yl group, 5-pyrimidyl group, pyrazyl group, 1,3,5-triazyl group, 4,6-diphenyl-1,3,5-triazin-2-yl group
(11): 1-benzimidazolyl group, 2-methyl-1-benzimidazolyl group, 2-phenyl-1-benzimidazolyl group, 1-methyl-2-benzimidazolyl group, 1-phenyl-2-benzimidazolyl group, 1-methyl-5-benzimidazolyl group, 1,2-dimethyl-5-benzimidazolyl group, 1-methyl-2-phenyl-5-benzimidazolyl group, 1-phenyl-5-benzimidazolyl group, 1,2-diphenyl-5-benzimidazolyl group, 1-methyl-6-benzimidazolyl group, 1,2-dimethyl-6-benzimidazolyl group, 1-methyl-2-phenyl-6-benzimidazolyl group, 1-phenyl-6-benzimidazolyl group, 1,2-diphenyl-6-benzimidazolyl group, 1-methyl -3-indazolyl group, 1-phenyl-3-indazolyl group
(12): 2-benzothiazolyl group, 4-benzothiazolyl group, 5-benzothiazolyl group, 6-benzothiazolyl group, 7-benzothiazolyl group, 3-benzoisothiazolyl group, 4-benzoisothiazolyl group, 5-benzoisothiazolyl group, 6-benzisothiazolyl group, 7-benzisothiazolyl group, 2,1,3-benzothiadiazol-4-yl group, 2,1,3-benzothiadiazol-5-yl group
(13): 2-benzoxazolyl group, 4-benzoxazolyl group, 5-benzoxazolyl group, 6-benzoxazolyl group, 7-benzoxazolyl group, 3-benzoisoxazolyl group, 4-benzisoxazolyl group, 5-benzisoxazolyl group, 6-benzisoxazolyl group, 7-benzisoxazolyl group, 2,1,3-benzoxadiazolyl-4-yl group, 2,1,3-benzoxadiazolyl-5-yl group
(14): 2-quinolyl group, 3-quinolyl group, 5-quinolyl group, 6-quinolyl group, 1-isoquinolyl group, 4-isoquinolyl group, 5-isoquinolyl group, 2-quinoxalyl group, 3-phenyl-2-quinoxalyl group, 6 -quinoxalyl group, 2,3-dimethyl-6-quinoxalyl group, 2,3-diphenyl-6-quinoxalyl group, 2-quinazolyl group, 4-quinazolyl group, 2-acridinyl group, 9-acridinyl group, 1,10-phenanthrolin-3-yl group, 1,10-phenanthrolin-5-yl group
(15): 2-thienyl group, 3-thienyl group, 2-benzothienyl group, 3-benzothienyl group, 2-dibenzothienyl group, 4-dibenzothienyl group
(16): 2-furanyl group, 3-furanyl group, 2-benzofuranyl group, 3-benzofuranyl group, 2-dibenzofuranyl group, 4-dibenzofuranyl group
(17): 9-methylcarbazol-2-yl group, 9-methylcarbazol-3-yl group, 9-methylcarbazol-4-yl group, 9-phenylcarbazol-2-yl group, 9-phenylcarbazol-3-yl group, 9-phenylcarbazol-4-yl group, 9-biphenylcarbazol-2-yl group, 9-biphenylcarbazol-3-yl group, 9-biphenylcarbazol-4-yl group
(18): 2-thianthryl group, 10-phenylphenothiazin-3-yl group, 10-phenylphenothiazin-2-yl group, 10-phenylphenoxazin-3-yl group, 10-phenylphenothiazin-2-yl group
(19): 1-methylindol-2-yl group, 1-phenylindol-2-yl group, 9-phenylcarbazol-4-yl group
(20): 4-(2-pyridyl)phenyl group, 4-(3-pyridyl)phenyl group, 4-(4-pyridyl)phenyl group, 3-(2-pyridyl)phenyl group, 3-(3-pyridyl)phenyl group, 3-(4-pyridyl)phenyl group
(21): 4-(2-phenylimidazol-1-yl) phenyl group, 4-(1-phenylimidazol-2-yl) phenyl group, 4-(2,3,4-triphenylimidazol-1-yl) phenyl group, 4-(1-methyl-4,5-diphenylimidazol-2-yl) phenyl group, 4-(2-methylbenzimidazol-1-yl) phenyl group, 4-(2-phenylbenzimidazol-1-yl) phenyl group, 4-(1-methylbenzimidazol-2-yl) phenyl group, 4-(2-phenylbenzimidazol-1-yl) phenyl group, 3-(2-methylbenzimidazol-1-yl) phenyl group, 3-(2-phenylbenzimidazol-1-yl) phenyl group, 3-(1-methylbenzimidazol-2-yl) phenyl group, 3-(1-phenylbenzimidazol-1-yl) phenyl group
(22): 4-(3,5-diphenyltriazin-1-yl)phenyl group, 4-(2-thienyl)phenyl group, 4-(2-furanyl)phenyl group, 5-phenylthiophen-2-yl group, 5-phenylfuran-2-yl group, 4-(5-phenylthiophen-2-yl) phenyl group, 4-(5-phenylfuran-2-yl) phenyl group, 3-(5-phenylthiophen-2-yl) phenyl group, 3-(5-phenylfuran-2-yl) phenyl group, 4-(2-benzothienyl) phenyl group, 4-(3-benzothienyl) phenyl group, 3-(2-benzothienyl) phenyl group, 3-(3-benzothienyl)phenyl group, 4-(2-dibenzothienyl) phenyl group, 4-(4-dibenzothienyl) phenyl group, 3-(2-dibenzothienyl) phenyl group, 3-(4-dibenzothienyl) phenyl group, 4-(2-dibenzofuranyl) phenyl group, 4-(4-dibenzofuranyl) phenyl group, 3-(2-dibenzofuranyl) phenyl group, 3-(4-dibenzofuranyl) phenyl group, 5-phenylpyridin-2-yl group, 4-phenylpyridin-2-yl group, 5-phenylpyridin-3-yl group, 4-(9-carbazolyl)phenyl group, 3-(9-carbazolyl)phenyl group
(23): 2-dibenzo[g,p]chrysenyl group, 3-dibenzo[g,p]chrysenyl group, 2-(7-phenyl)dibenzo[g,p]chrysenyl group, 3-(7-phenyl)dibenzo[g,p ] chrysenyl group
(24): N,N-diphenylamino group, N,N-bis(4-biphenylyl)-amino group, N,N-bis(3-biphenylyl)-amino group, N-phenyl-4-biphenylamino group, N- phenyl-3-biphenylamino group, N-(4-biphenyl)-4-p-terphenylamino group, N-[4-(carbazol-9-yl)phenyl]-4-biphenylamino group, N3-[1 , 1'-biphenyl]-4-yl-N1,N1-diphenyl-1,3-benzenediamino group, 4-triphenylamino group, 3-triphenylamino group, 4-(4',4' '-diphenyl) triphenylamino group, 3-(4',4''-diphenyl)triphenylamino group, N1,N1,N3,N3-tetraphenyl-1,3-benzenediamino group, 4-(phenylamino)triphenyl amino group

In the condensed ring compounds represented by Formulas (2A) to (5A), in the point of ease of material obtaining, A is each independently a phenyl group, biphenyl group, pyridylphenyl group, terphenylyl group, naphthyl group, phenanthryl group, pyrenyl group, 9,9-spirobi[9H-fluorenyl] group, triphenylenyl group, dibenzothienyl group, dibenzofuranyl group, pyridyl group, pyrimidyl group, or group in which these groups are substituted by a cyano group, nitro group, hydroxy group, thiol group, fluorine atom, chlorine atom, bromine atom, iodine atom, methyl group, methoxy group or trifluoromethylsulfonyloxy group;
a fluorenyl group, benzofluorenyl group, anthryl group, dibenzo[g,p]chrysenyl group, carbazolyl group, or group in which these groups are substituted by a cyano group, nitro group, hydroxy group, thiol group, fluorine atom, chlorine atom, bromine atom, iodine atom, methyl group, methoxy group or phenyl group;
a 4,6-diphenyl-1,3,5-triazin-2-yl group, (4,6-diphenyl-1,3,5-triazin-2-yl)phenyl group, 4,6-bis(4-biphenylyl-1,3,5-triazin-2-yl group, 4,6-bis(3-biphenylyl)-1,3,5-triazin-2-yl group, cyano group, nitro group, hydroxy group, thiol group, fluorine atom, chlorine atom, bromine atom, iodine atom, diphenylphosphine oxide, triphenylsilyl group, dihydroxyboryl group (-B(OH)₂), 4,4,5,5-tetramethyl-[1,3,2]-dioxaborolanyl group, 5,5-dimethyl-[1,3,2]-dioxaborinane group, methyl group, N,N-diphenylamino group, N,N-bis(4-biphenylyl)amino group, N3-[1,1'-biphenyl ]-4-yl-N1,N1-diphenyl-1,3-benzenediamino group, N-phenyl-3-biphenylylamino group, 4-triphenylamino group, 3-triphenylamino group, 4-(4',4''-diphenyl)triphenylamino group, 3-(4',4"-diphenyl) triphenylamino group, N¹,N¹,N³,N³-tetraphenyl-1,3-benzenediamino group, or 4-(phenylamino)triphenylamino group.

### <Preferred Specific Examples of Photoelectric Conversion Element Material for Imaging Elements>

Hereinafter, preferred specific examples are shown for the photoelectric conversion element material for imaging elements represented by Formula (1); however, it is not to be limited thereto.

Tables B-1 to B-7 show compounds (N-1) to (N-275) having the backbone of (3Aa) to (4Cd) and substitution sites shown in Tables A-1 to A-3, and the substituents A of these substitution sites are the groups shown in Tables B-1 to B-7.
Herein, N represents any symbol of 3Aa to 4Cd. Therefore, for example, in the case of a compound such as (3Aa-3), when N=3Aa indicates the compound of (3Aa-3) having the backbone of (3Aa) and in which the substituent having this backbone is the Cl atom.

**[Table A-1]**

| | | | |
|---|---|---|---|
| 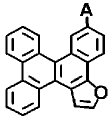 | 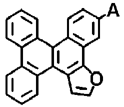 | 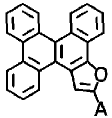 | |
| 3 A a | 3 A b | 3 A c | |
| 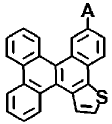 | 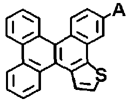 | 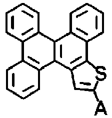 | |
| 3 B a | 3 B b | 3 B c | |
| 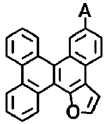 | 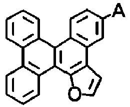 | 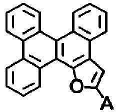 | |
| 3 C a | 3 C b | 3 C c | |
| 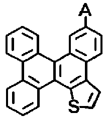 | 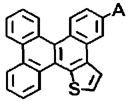 | 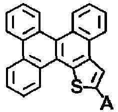 | |
| 3 D a | 3Db | 3 D c | |
| 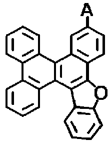 | 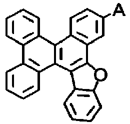 | 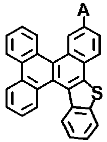 | 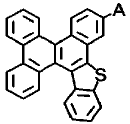 |
| 3 E a | 3 E b | 3 F c | 3 F b |
| 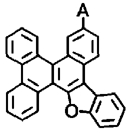 | 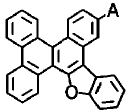 | 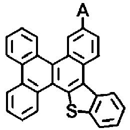 | 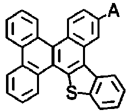 |
| 3 G a | 3 G b | 3 H a | 3 H b |
| 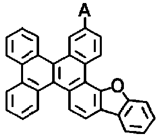 | 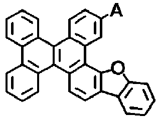 | 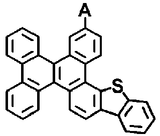 | 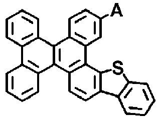 |
| 3 I a | 3 I b | 3 J a | 3 J b |

**[Table A-2]**

| | | | |
|---|---|---|---|
| 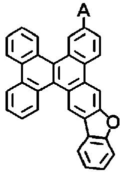 | 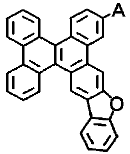 | 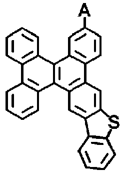 | 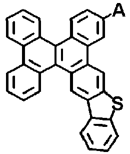 |
| 3 K a | 3 K b | 3 L a | 3 L b |
| 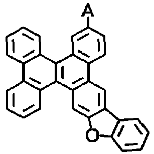 | 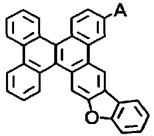 | 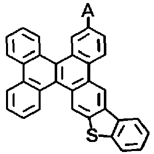 | 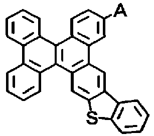 |
| 3 M a | 3 M b | 3 N a | 3 N b |
| 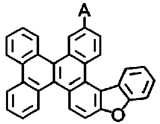 | 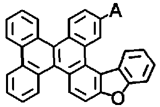 | 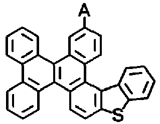 | 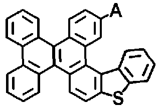 |
| 3 O a | 3 O b | 3 P a | 3 P b |

**[Table A-3]**

| | | | |
|---|---|---|---|
| 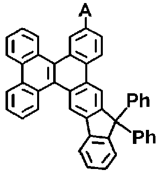 | 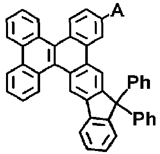 | 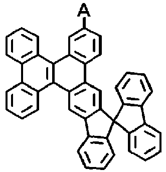 | 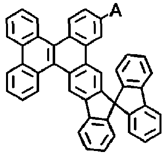 |
| 4 A a | 4 A b | 4 A c | 4 A d |
| 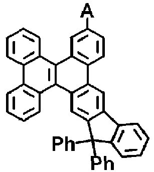 | 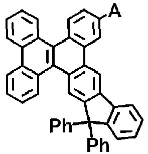 | 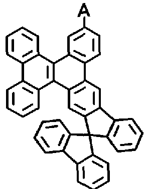 | 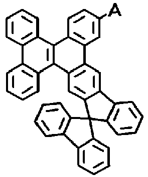 |
| 4 B a | 4 B b | 4 B c | 4 B d |
| 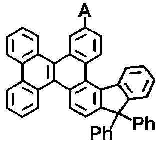 | 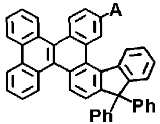 | 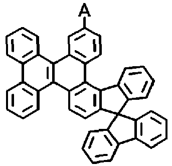 | 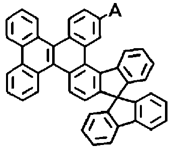 |
| 4 C a | 4 C b | 4 C c | 4 C d |

**[Table B-1]**

| Formula | (1A-m) · · · (1F-m) | | | | |
|---|---|---|---|---|---|
| A | **---D** | **---F** | **---Cl** | **---Br** | **---I** |
| m | 1 | 2 | 3 | 4 | 5 |
| A | 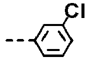 | 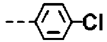 | 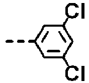 | 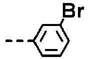 | 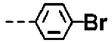 |
| m | 6 | 7 | 8 | 9 | 10 |
| A | 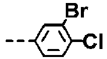 | **---Me** | **---Et** | **----*ⁱ*Pr** | **---*ⁿ*Bu** |
| m | 11 | 12 | 13 | 14 | 15 |
| A | **---*^{t}*Bu** | **---*ⁿ*hex** | **---OMe** | 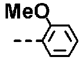 | 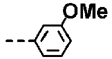 |
| m | 16 | 17 | 18 | 19 | 20 |
| A | 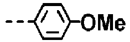 | 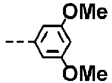 | 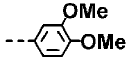 | **---OH** | 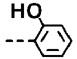 |
| m | 21 | 22 | 23 | 24 | 25 |
| A | 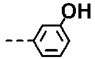 | 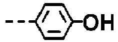 | 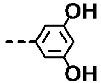 | 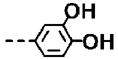 | **---OTf** |
| m | 26 | 27 | 28 | 29 | 30 |
| A | 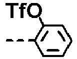 | 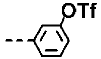 | 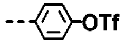 | 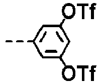 | 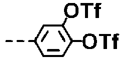 |
| m | 31 | 32 | 33 | 34 | 35 |
| A | **---CF₃** | **---NO₂** | **---CN** | 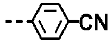 | 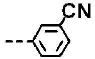 |
| m | 36 | 37 | 38 | 39 | 40 |

**[Table B-2]**

| Formula | (1A-m) · · · (1F-m) | | | | |
|---|---|---|---|---|---|
| A | 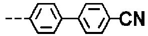 | 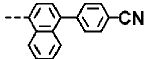 | 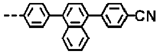 | 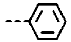 | 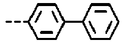 |
| m | 41 | 42 | 43 | 44 | 45 |
| A | 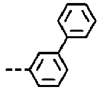 | 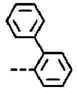 | 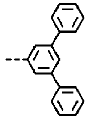 | 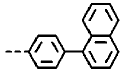 | 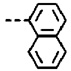 |
| m | 46 | 47 | 48 | 49 | 50 |
| A | 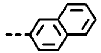 | 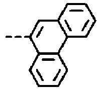 | 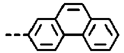 | 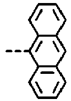 | 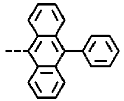 |
| m | 51 | 52 | 53 | 54 | 55 |
| A | 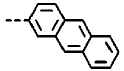 | 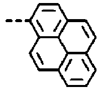 | 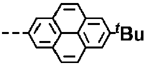 | 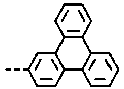 | 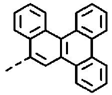 |
| m | 56 | 57 | 58 | 59 | 60 |
| A | 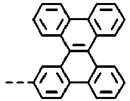 | 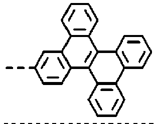 | 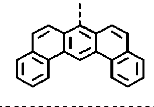 | 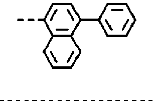 | 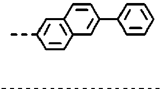 |
| m | 61 | 62 | 63 | 64 | 65 |
| A | 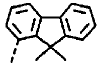 | 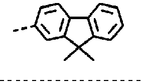 | 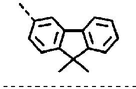 | 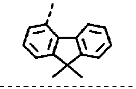 | 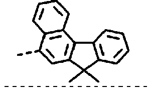 |
| m | 66 | 67 | 68 | 69 | 70 |
| A | 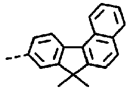 | 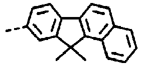 | 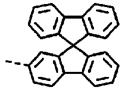 | 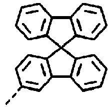 | 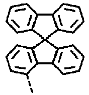 |
| m | 71 | 72 | 73 | 74 | 75 |
| A | 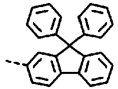 | 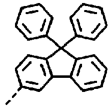 | 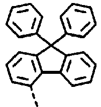 | 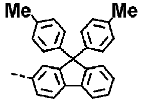 | 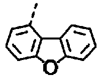 |
| m | 76 | 77 | 78 | 79 | 80 |

**[Table B-3]**

| Formula | (1A-m) · · · (1F-m) | | | | |
|---|---|---|---|---|---|
| A | 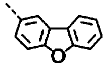 | 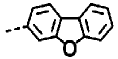 | 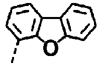 | 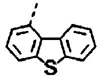 | 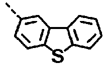 |
| m | 81 | 82 | 83 | 84 | 85 |
| A | 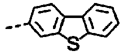 | 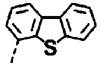 | 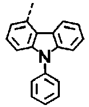 | 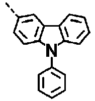 | 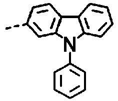 |
| m | 86 | 87 | 88 | 89 | 90 |
| A | 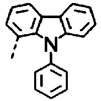 | 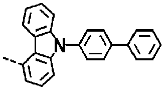 | 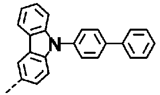 | 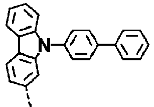 | 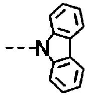 |
| m | 91 | 92 | 93 | 94 | 95 |
| A | 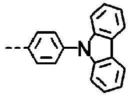 | 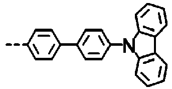 | 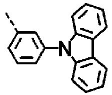 | 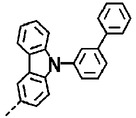 | 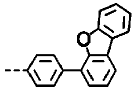 |
| m | 96 | 97 | 98 | 99 | 100 |
| A | 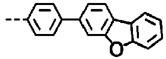 | 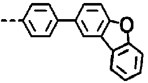 | 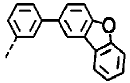 | 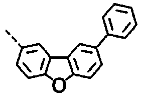 | 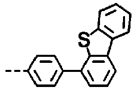 |
| m | 101 | 102 | 103 | 104 | 105 |
| A | 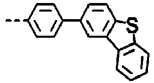 | 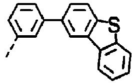 | 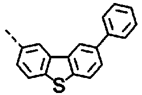 | 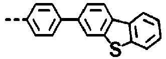 | 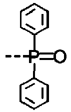 |
| m | 106 | 107 | 108 | 109 | 110 |
| A | 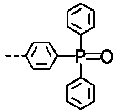 | 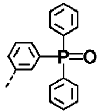 | 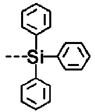 | 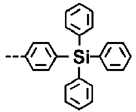 | 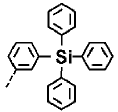 |
| m | 111 | 112 | 113 | 114 | 115 |
| A | 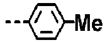 | 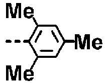 | 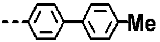 | 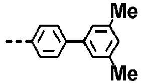 | 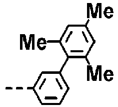 |
| m | 116 | 117 | 118 | 119 | 120 |

**[Table B-4]**

| Formula | (1A-m) · · · (1F-m) | | | | |
|---|---|---|---|---|---|
| A | 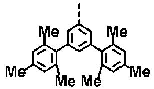 | 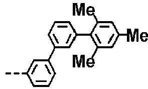 | 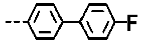 | 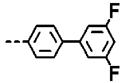 | 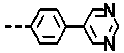 |
| m | 121 | 122 | 123 | 124 | 125 |
| A | 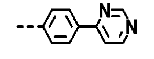 | 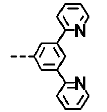 | 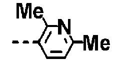 | 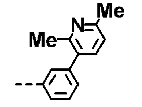 | 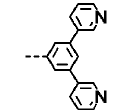 |
| m | 126 | 127 | 128 | 129 | 130 |
| A | 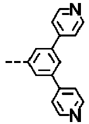 | 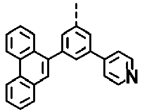 | 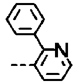 | 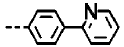 | 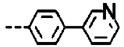 |
| m | 131 | 132 | 133 | 134 | 135 |
| A | 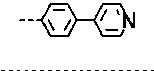 | 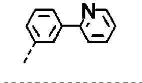 | 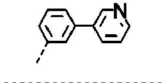 | 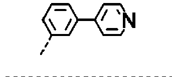 | 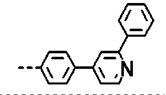 |
| m | 136 | 137 | 138 | 139 | 140 |
| A | 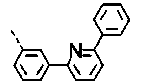 | ---B(OH)₂ | 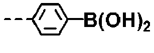 | 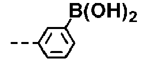 | 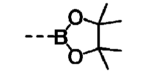 |
| m | 141 | 142 | 143 | 144 | 145 |
| A | 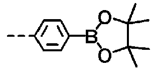 | 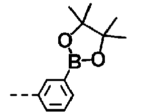 | 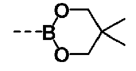 | 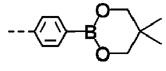 | 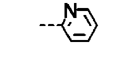 |
| m | 146 | 147 | 148 | 149 | 150 |
| A | 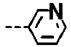 | 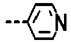 | 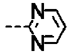 | 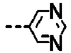 | 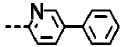 |
| m | 151 | 152 | 153 | 154 | 155 |
| A | 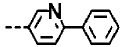 | 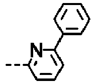 | 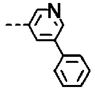 | 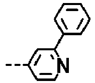 | 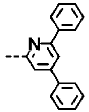 |
| m | 156 | 157 | 158 | 159 | 160 |

**[Table B-5]**

| Formula | (1A-m) · · · (1F-m) | | | | |
|---|---|---|---|---|---|
| A | 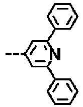 | 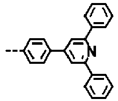 | 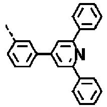 | 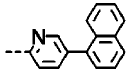 | 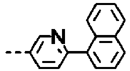 |
| m | 161 | 162 | 163 | 164 | 165 |
| A | 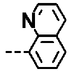 | 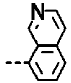 | 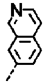 | 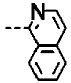 | 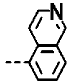 |
| m | 166 | 167 | 168 | 169 | 170 |
| A | 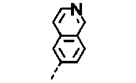 | 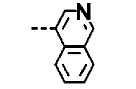 | 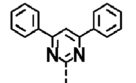 | 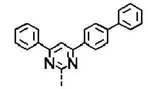 | 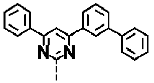 |
| m | 171 | 172 | 173 | 174 | 175 |
| A | 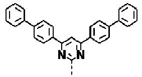 | 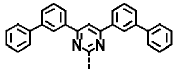 | 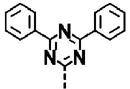 | 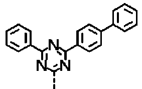 | 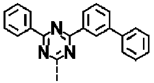 |
| m | 176 | 177 | 178 | 179 | 180 |
| A | 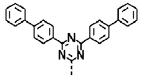 | 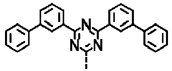 | 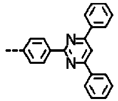 | 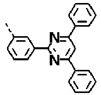 | 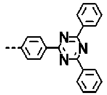 |
| m | 181 | 182 | 183 | 184 | 185 |
| A | 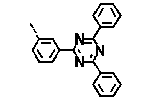 | 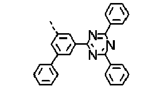 | 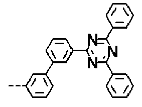 | 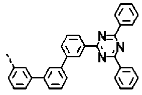 | 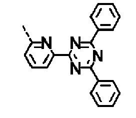 |
| m | 186 | 187 | 188 | 189 | 190 |
| A | 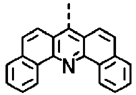 | 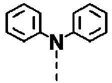 | 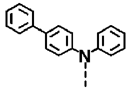 | 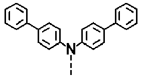 | 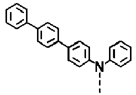 |
| m | 191 | 192 | 193 | 194 | 195 |
| A | 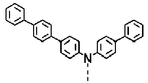 | 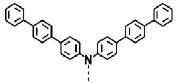 | 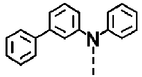 | 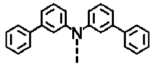 | 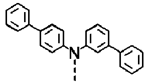 |
| m | 196 | 197 | 198 | 199 | 200 |

**[Table B-6]**

| Formula | (1A-m) · · · (1F-m) | | | | |
|---|---|---|---|---|---|
| A | 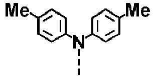 | 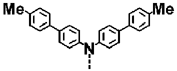 | 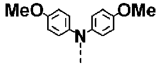 | 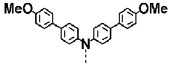 | 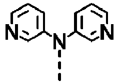 |
| m | 201 | 202 | 203 | 204 | 205 |
| A | 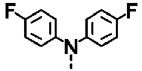 | 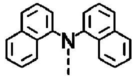 | 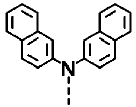 | 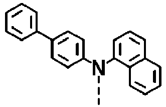 | 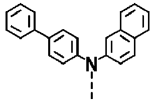 |
| m | 206 | 207 | 208 | 209 | 210 |
| A | 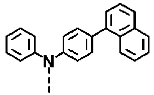 | 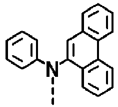 | 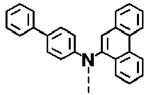 | 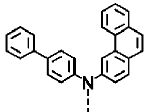 | 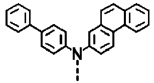 |
| m | 211 | 212 | 213 | 214 | 215 |
| A | 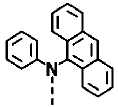 | 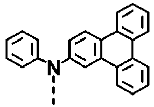 | 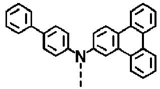 | 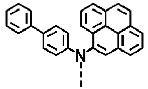 | 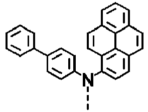 |
| m | 216 | 217 | 218 | 219 | 220 |
| A | 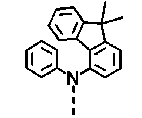 | 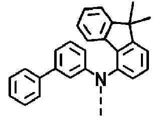 | 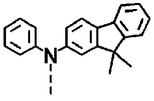 | 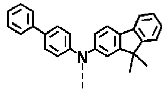 | 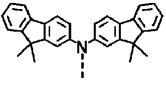 |
| m | 221 | 222 | 223 | 224 | 225 |
| A | 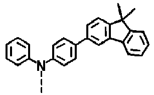 | 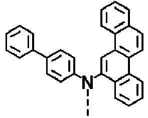 | 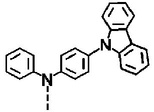 | 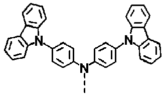 | 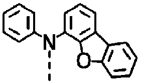 |
| m | 226 | 227 | 228 | 229 | 230 |
| A | 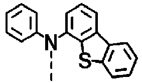 | 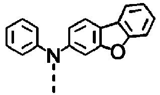 | 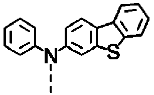 | 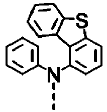 | 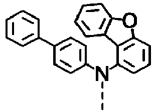 |
| m | 231 | 232 | 233 | 234 | 235 |
| A | 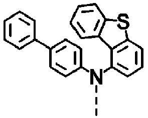 | 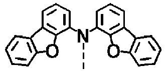 | 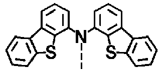 | 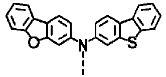 | 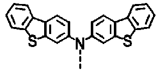 |
| m | 236 | 237 | 238 | 239 | 240 |

**[Table B-7]**

| Formula | (1A-m) · · · (1F-m) | | | | |
|---|---|---|---|---|---|
| A | 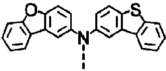 | 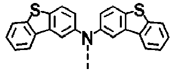 | 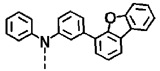 | 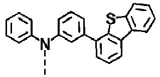 | 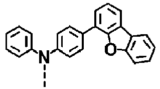 |
| m | 241 | 242 | 243 | 244 | 245 |
| A | 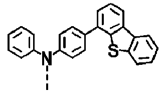 | 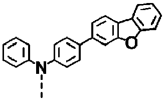 | 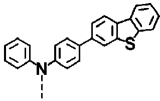 | 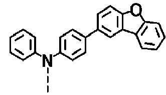 | 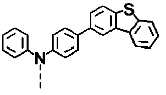 |
| m | 246 | 247 | 248 | 249 | 250 |
| A | 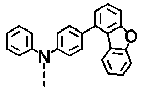 | 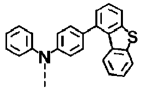 | 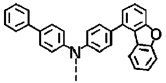 | 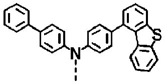 | 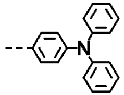 |
| m | 251 | 252 | 253 | 254 | 255 |
| A | 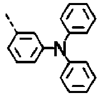 | 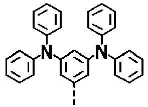 | 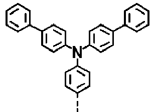 | 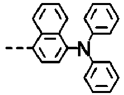 | 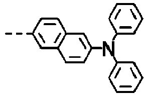 |
| m | 256 | 257 | 258 | 259 | 260 |
| A | 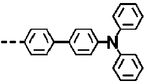 | 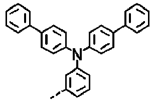 | 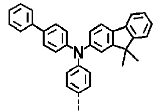 | 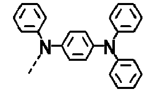 | 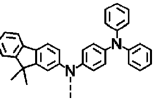 |
| m | 261 | 262 | 263 | 264 | 265 |
| A | 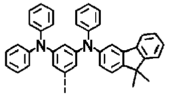 | 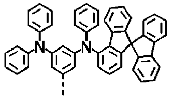 | 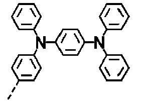 | 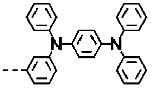 | 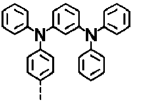 |
| m | 266 | 267 | 268 | 269 | 270 |
| A | 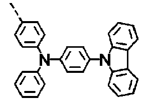 | 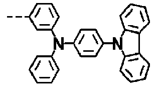 | 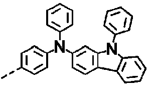 | 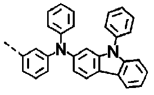 | 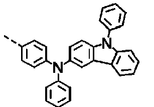 |
| m | 271 | 272 | 273 | 274 | 275 |

For the preferred specific examples of the photoelectric conversion element material for imaging elements represented by Formula (1), the compounds shown below are also included.

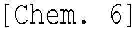

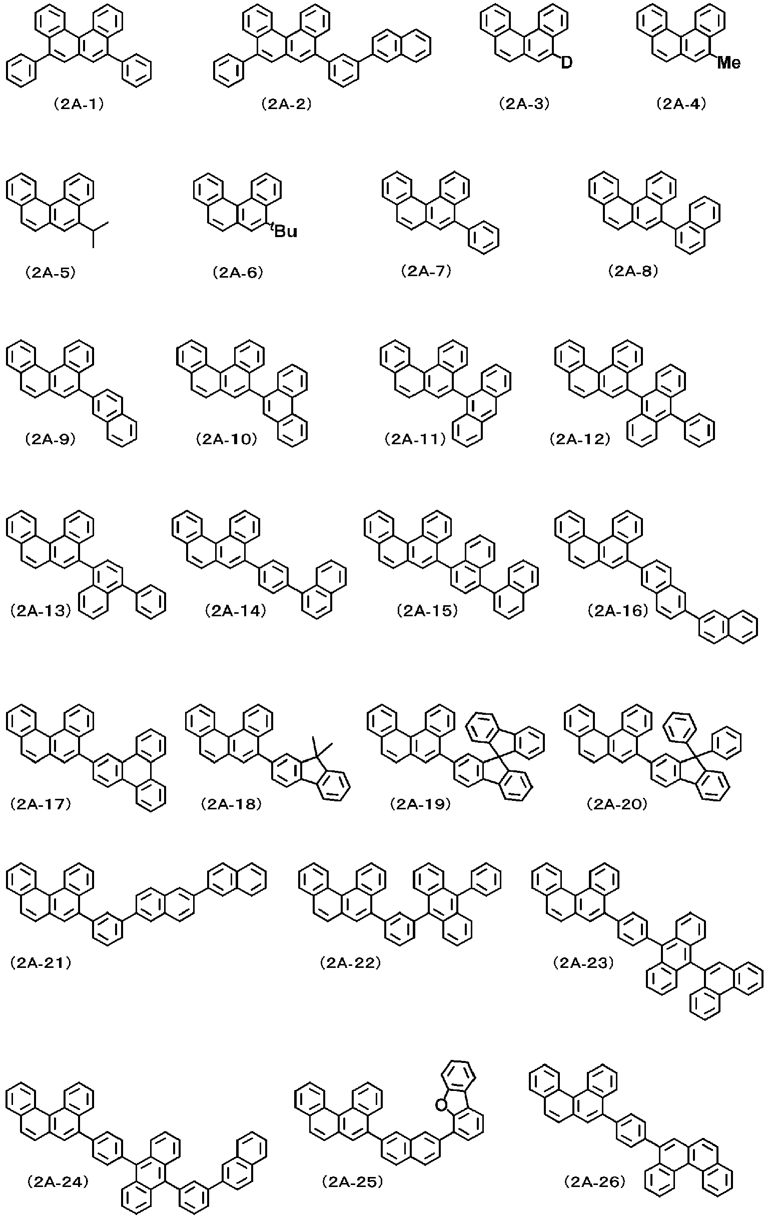

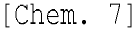

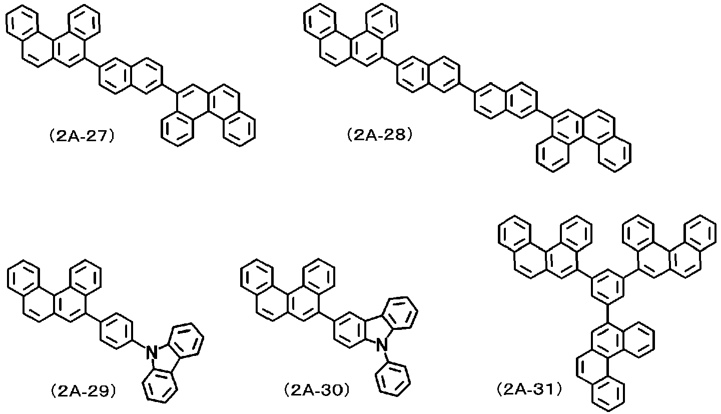

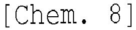

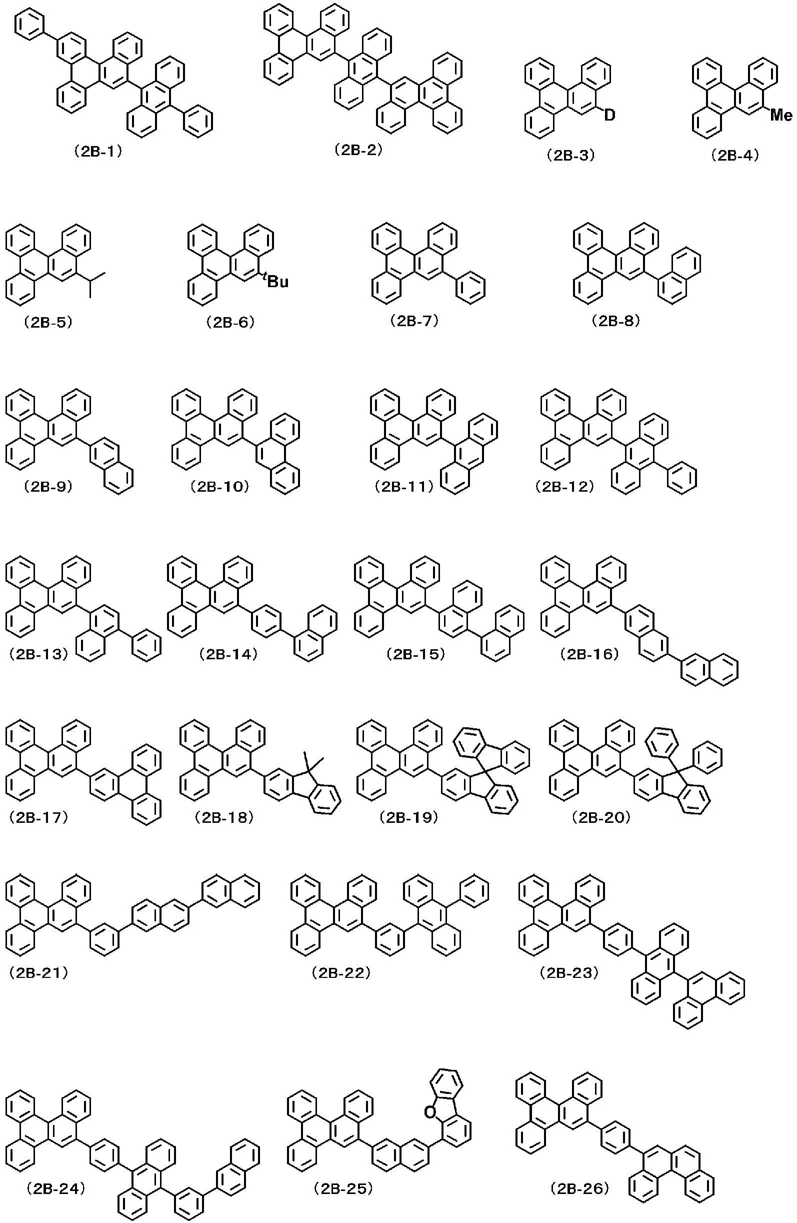

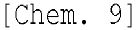

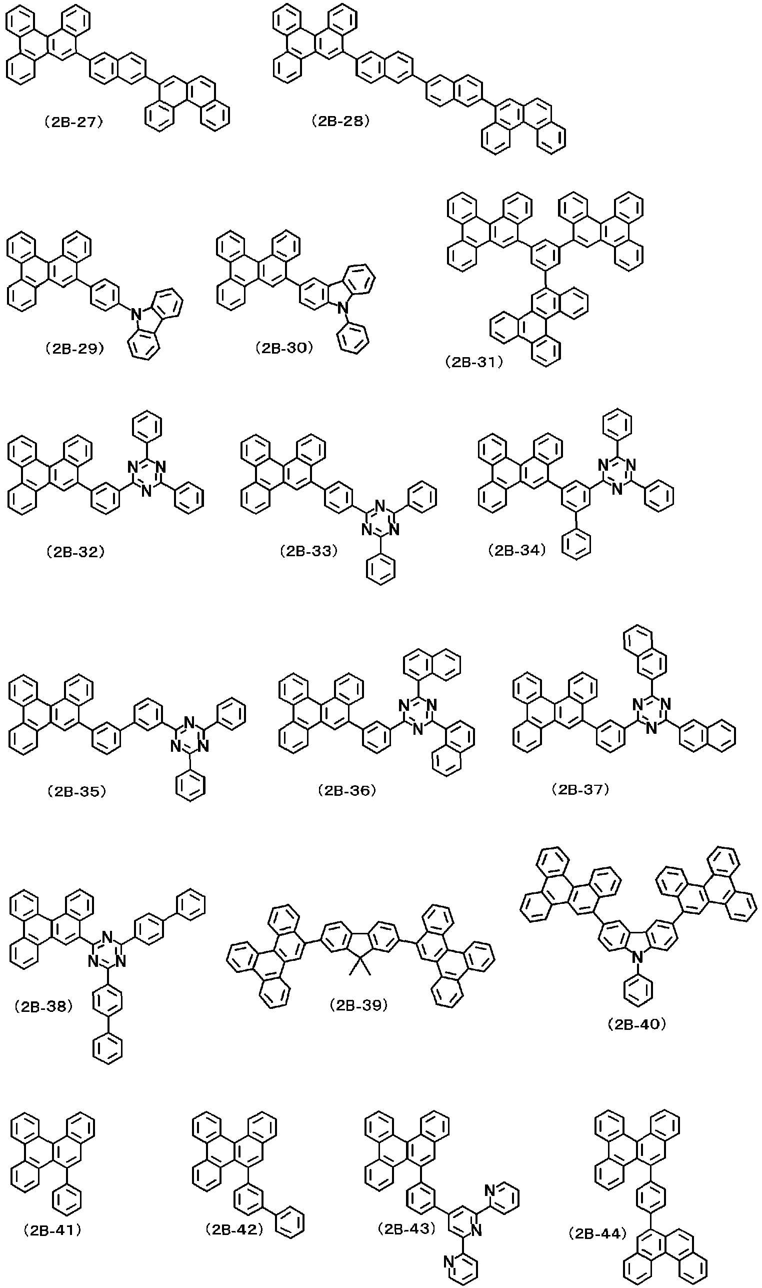

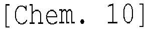

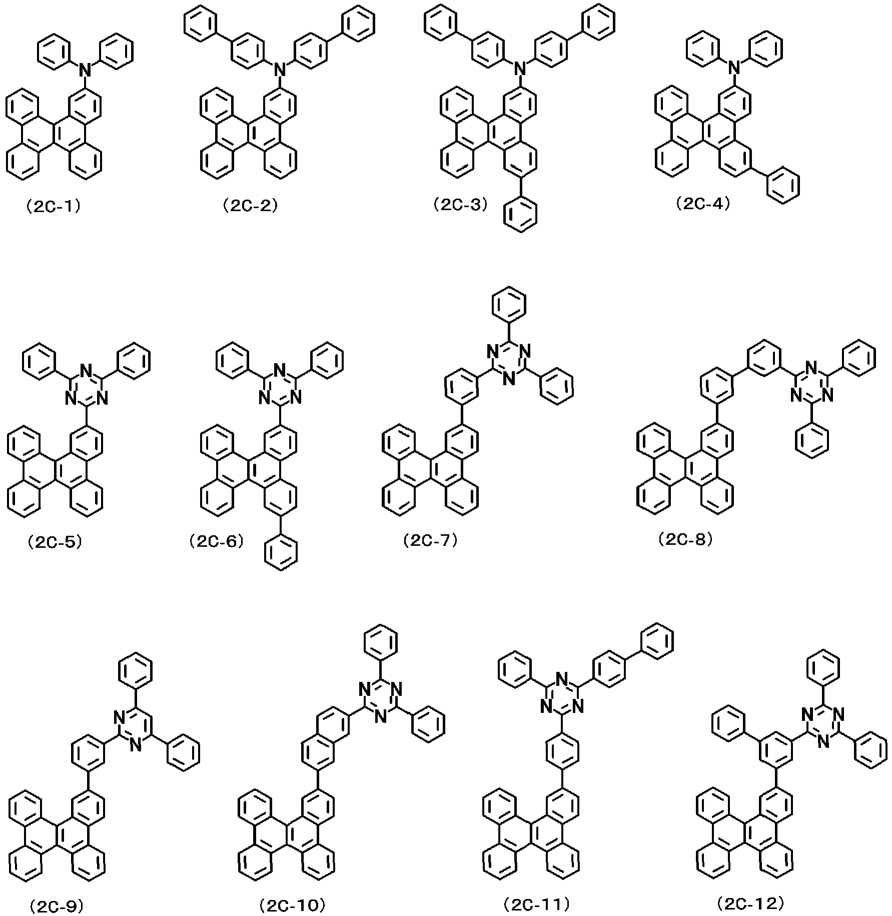

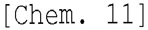

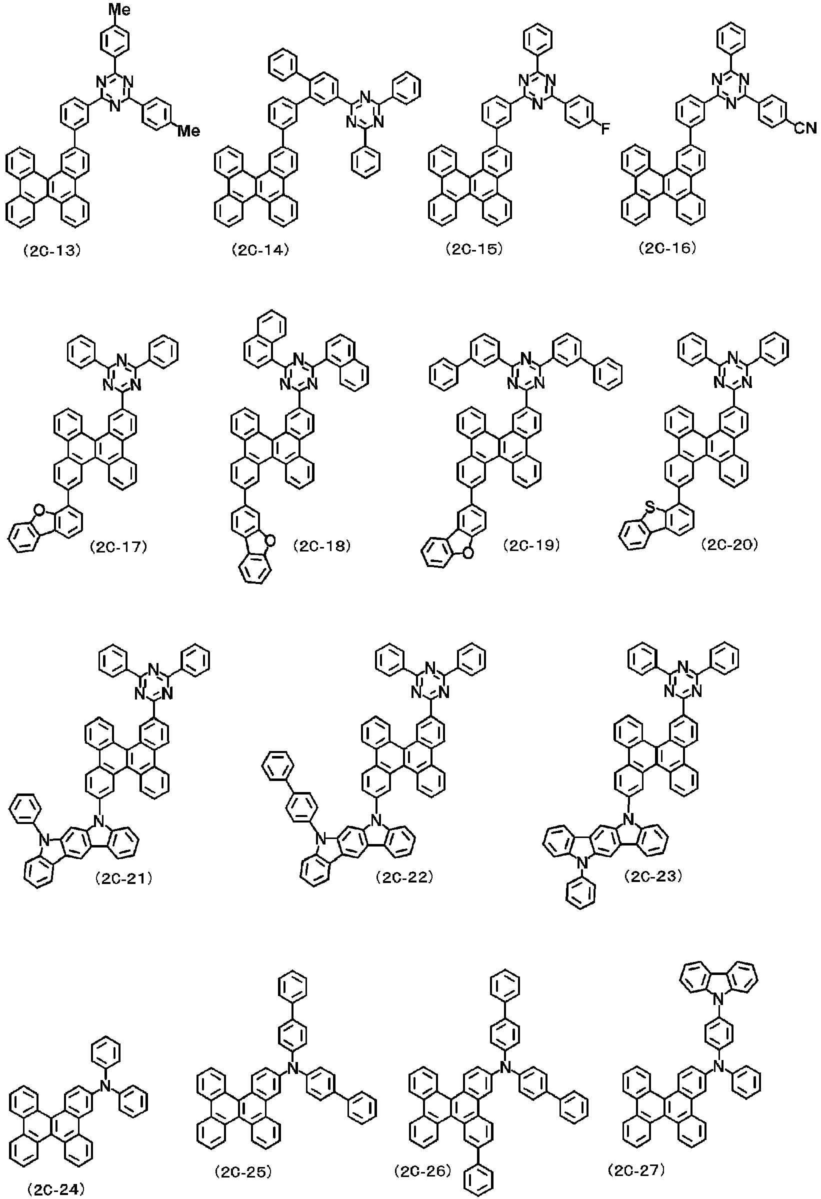

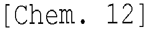

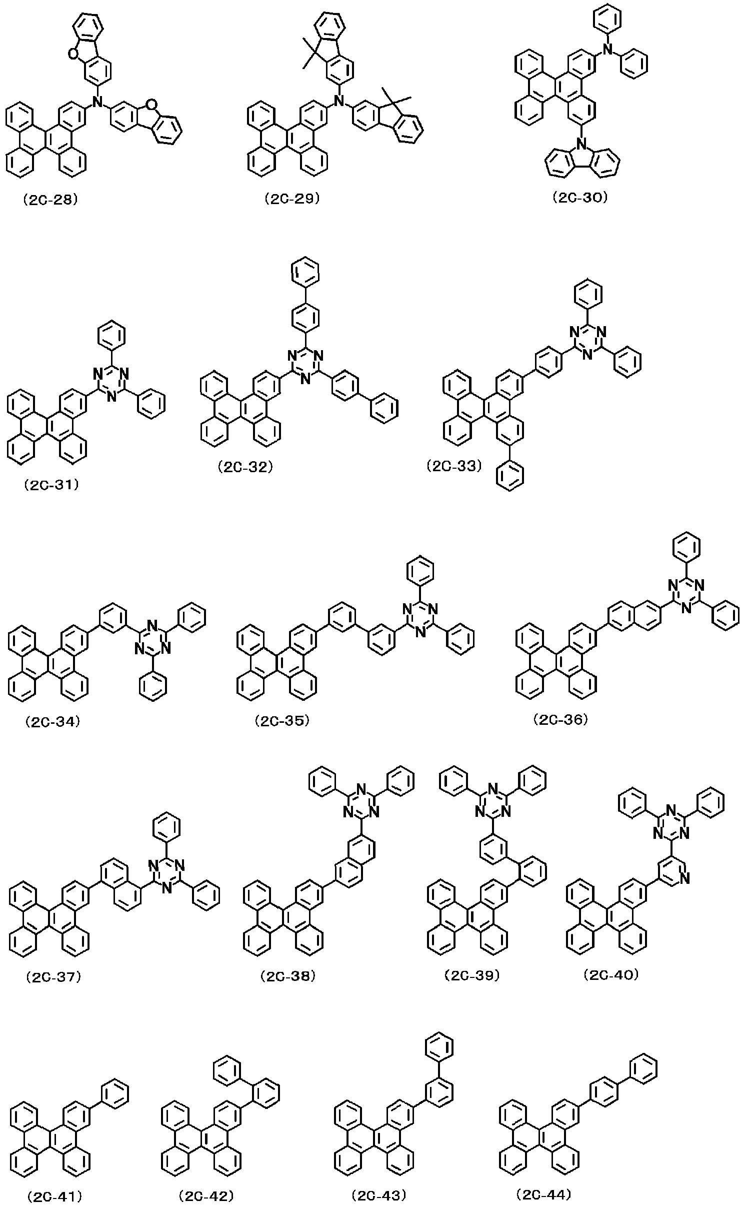

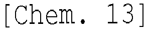

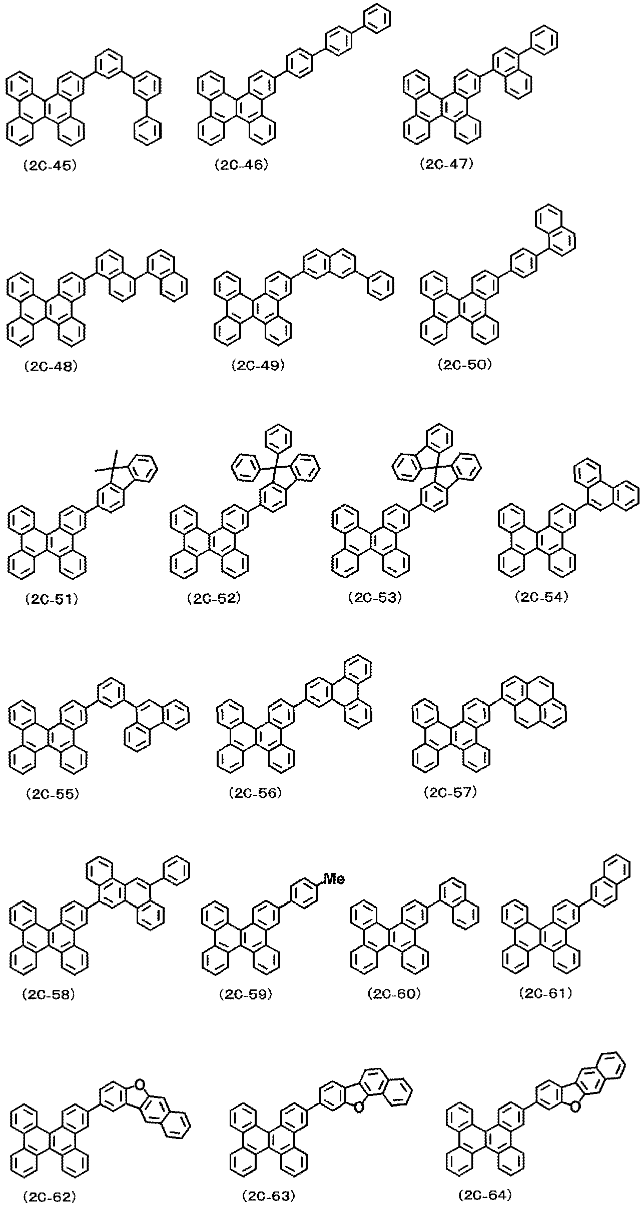

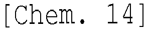

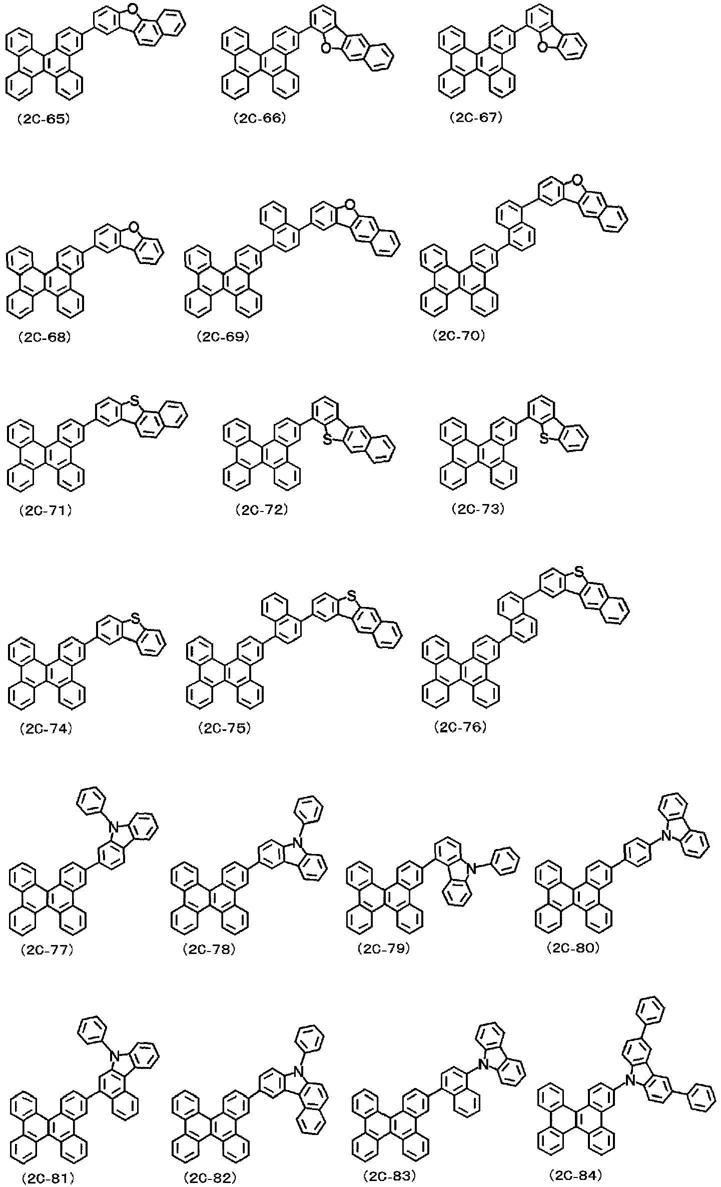

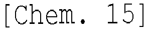

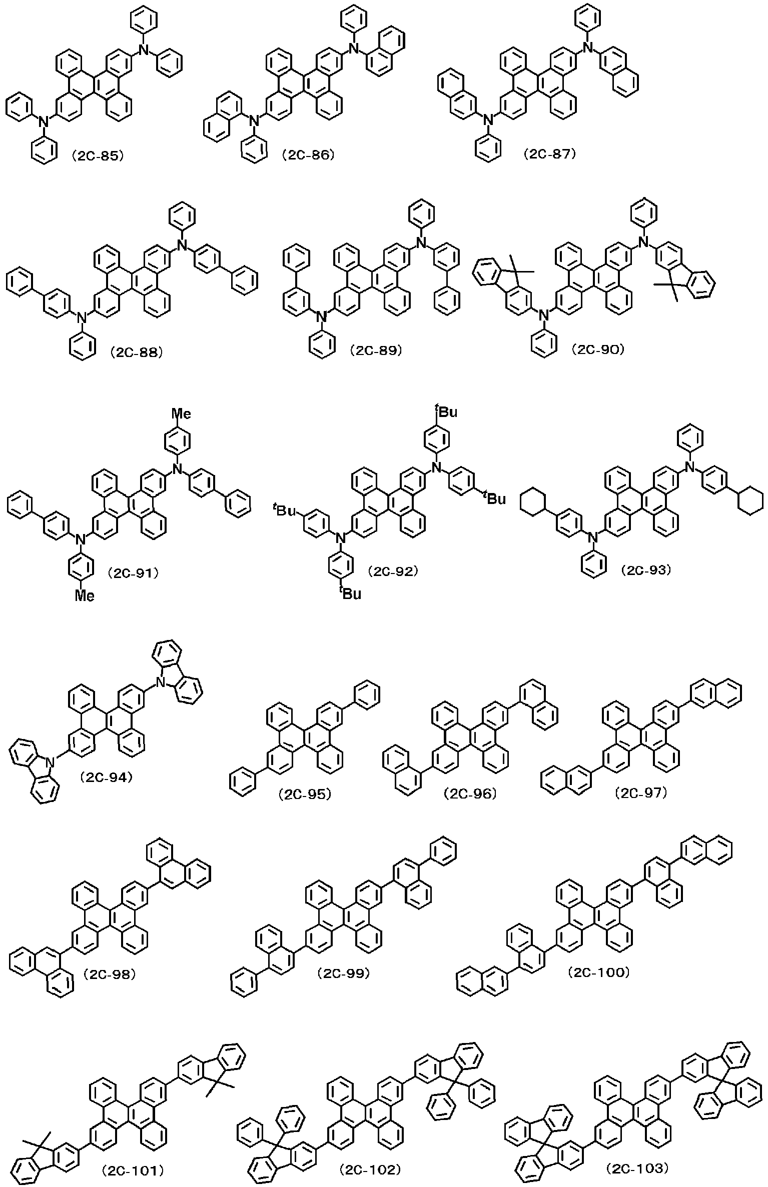

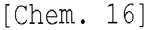

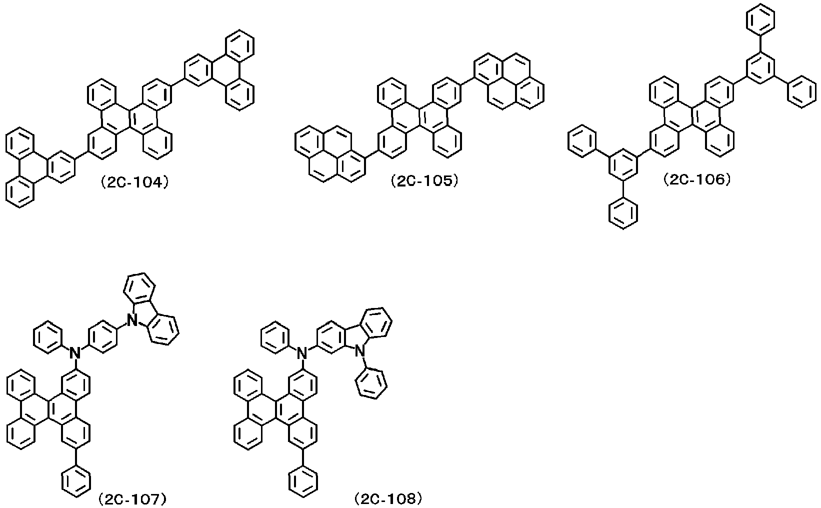

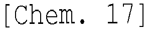

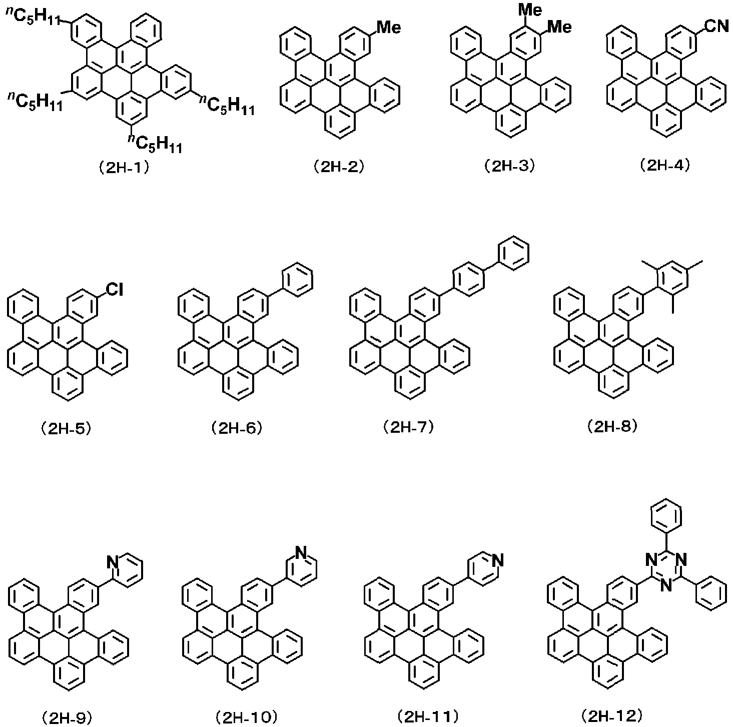

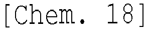

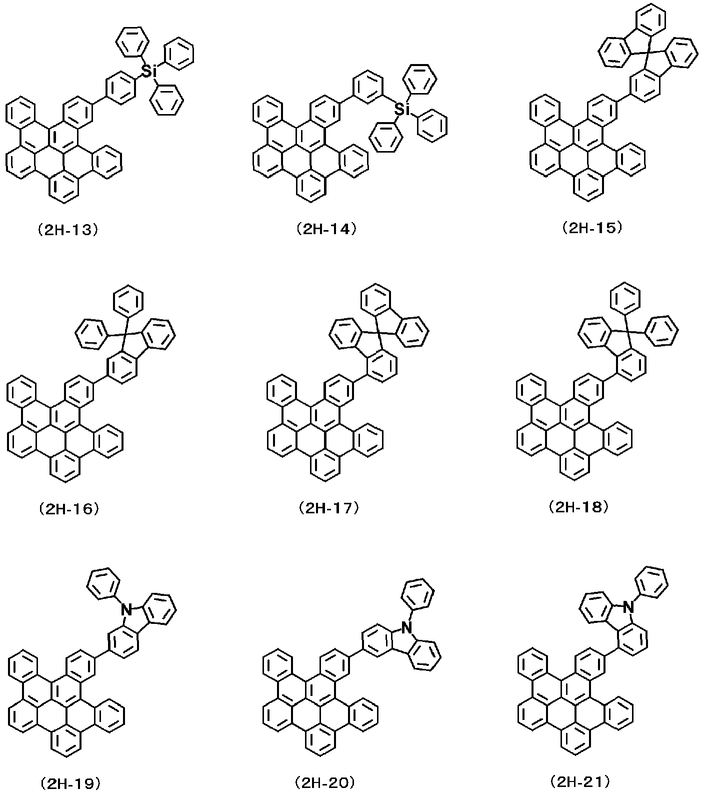

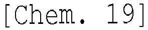

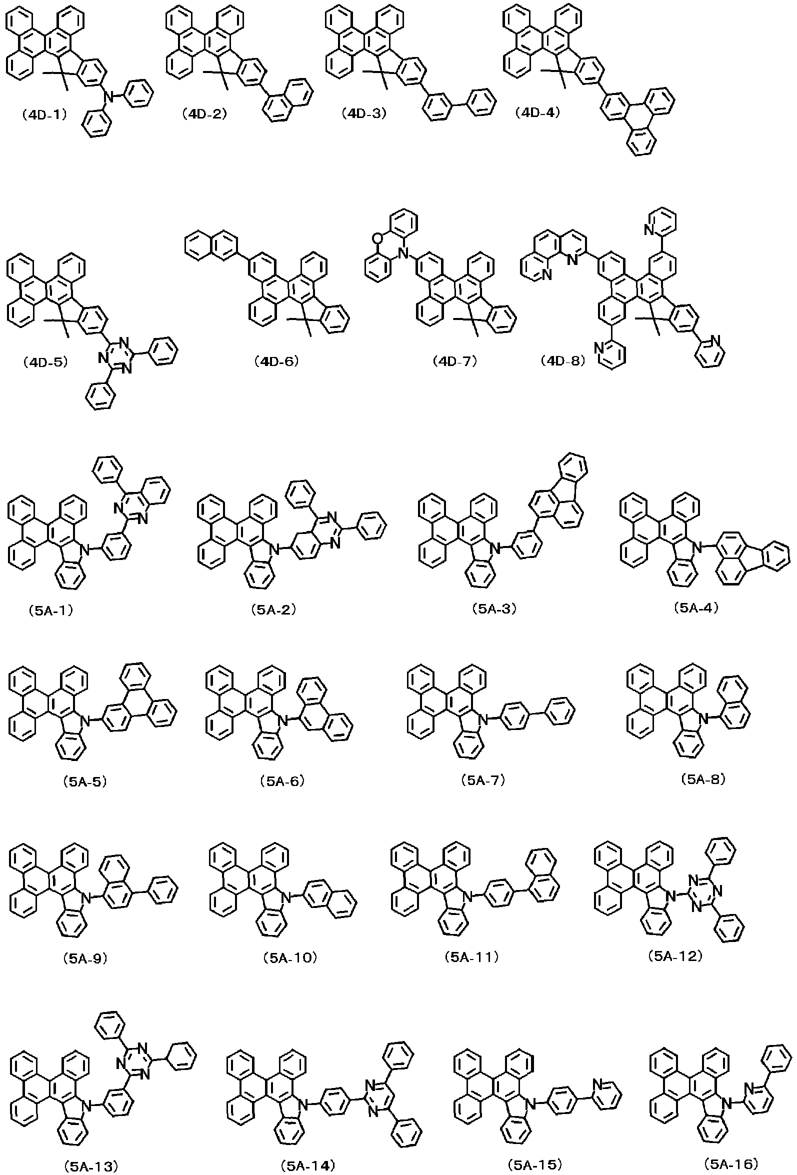

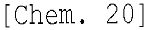

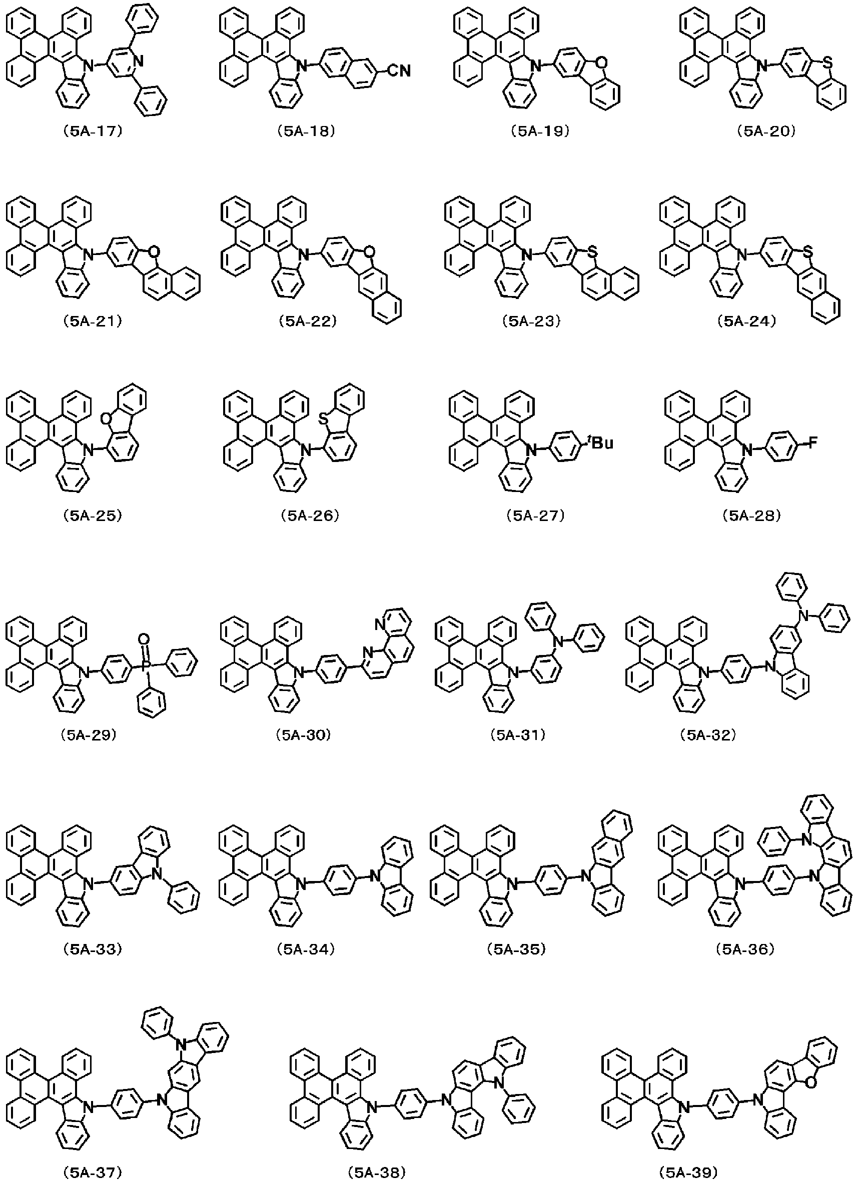

For the preferred specific examples of the photoelectric conversion element material for imaging elements represented by Formula (1), the compounds shown below are also included.

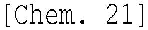

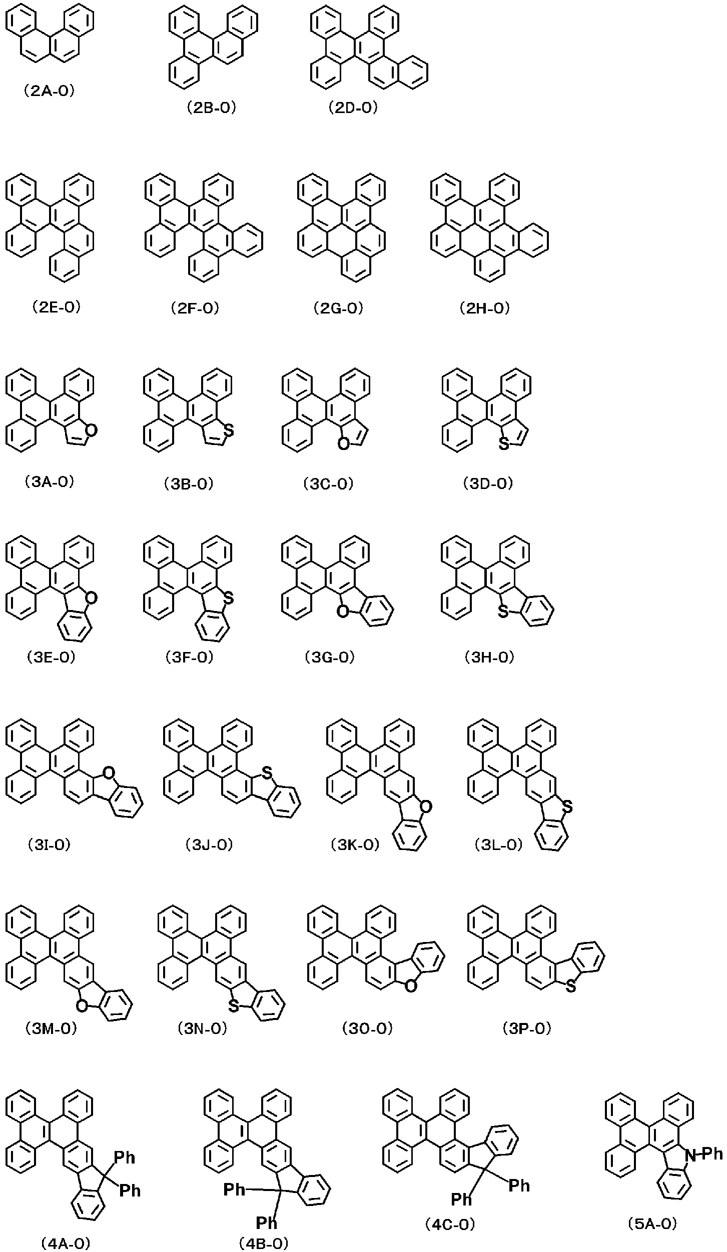

The photoelectric conversion element material for imaging elements according to an embodiment of the present invention can be synthesized by a known method. For example, it is possible to synthesize by a known method disclosed in Japanese Unexamined Patent Application, Publication No. 2018-193371; Japanese Unexamined Patent Application, Publication No. 2019-34939; Japanese Unexamined Patent Application, Publication No. 2019-116472; Japanese Unexamined Patent Application, Publication No. 2019-34933; Japanese Unexamined Patent Application, Publication No. 2020-33332; Japanese Unexamined Patent Application, Publication No. 2020-15691; Japanese Unexamined Patent Application, Publication No. 2011-6397; Japanese Patent No. 4968333; US Patent Application, Publication No. 2019/0198781; and PCT International Application, Publication No. WO2017/109722.

Hereinafter, application of the photoelectric conversion element material for imaging elements according to an embodiment of the present invention will be explained.

### <Photoelectric Conversion Element Material for Imaging Elements, and Photoelectric Conversion Element Transport Material for Imaging Elements>

A photoelectric conversion element material for imaging elements according to an aspect of the present invention can use a known material. The photoelectric conversion element material for imaging elements, for example, can be used as a photoelectric conversion element transport material for imaging elements, and a photoelectric conversion element charge blocking material for imaging elements.

A photoelectric conversion element material for imaging elements according to an aspect includes a compound containing the backbone represented by the above Formula (1). In addition, the photoelectric conversion element transport material for imaging elements and the photoelectric conversion element charge blocking material for imaging elements according to an aspect of the present invention includes a compound containing the backbone represented by Formula (1). The photoelectric conversion element material for imaging elements, the photoelectric conversion element transport material for imaging elements and the photoelectric conversion element charge blocking material for imaging elements including the compound containing the backbone represented by Formula (1) contribute to the production of a photoelectric conversion element material for imaging elements superior in dark current and external quantum efficiency characteristic.

### <Regarding Molecular Weight>

The photoelectric conversion element material for imaging elements according to an aspect of the present invention, or the photoelectric conversion element material for imaging elements shown by Formula (1) preferably has a molecular weight of 1500 or less, and more preferably 1000 or less, from the viewpoint of the heat resistance stability during sublimation.

### <Regarding HOMO Level>

The HOMO level of the photoelectric conversion element material for imaging elements according to an aspect of the present invention, or the photoelectric conversion element material for imaging elements shown by Formula (1) is not particularly limited; however, HOMO is preferably 5.0 to 6.5 eV from the viewpoint of compatibility to a photoelectric conversion element for imaging elements. It should be noted that this HOMO value is a value obtained from measurement by an atmospheric photoelectron yield spectrometer on a vapor deposited film.

### <Regarding Band Gap>

The band gap of the photoelectric conversion element material for imaging elements according to an aspect of the present invention, or the photoelectric conversion element material for imaging elements shown by Formula (1) is not particularly limited; however, the band gap is preferably 2.5 to 4.0 eV from the viewpoint of compatibility to photoelectric conversion element for imaging elements. It should be noted that this band gap is a value obtained from the wavelength edge of the absorption spectrum of the vapor deposited film.

### <Regarding LUMO Level>

The LUMO level of the photoelectric conversion element material for imaging elements according to an aspect of the present invention, or the photoelectric conversion element material for imaging elements shown by Formula (1) is not particularly limited; however, LUMO is preferably 2.0 to 3.5 eV from the viewpoint of compatibility to photoelectric conversion element for imaging elements. It should be noted that this LUMO value is a value obtained from the above-mentioned HOMO value and band gap.

### <Regarding Glass Transition Temperature>

The glass transition temperature of the photoelectric conversion element material for imaging elements according to an aspect of the present invention, or the photoelectric conversion element material for imaging elements shown by Formula (1) is not particularly limited; however, the glass transition temperature is preferably at least 100°C, more preferably at least 110°C, and particularly preferably at least 130°C from the viewpoint of compatibility to photoelectric conversion element for imaging elements. It should be noted that this glass transition temperature is a value obtained from differential scanning calorimetry.

### <Regarding Amorphous Property>

The photoelectric conversion element material for imaging elements according to an aspect of the present invention, or the photoelectric conversion element material for imaging elements shown by Formula (1) preferably form an amorphous layer by the vapor deposited film of this material. When the vapor deposited film is a crystalline layer, since the interface with an adjacent layer is not uniform, it will be a factor for defects in the element. The confirmation method for whether the vapor deposited film is an amorphous layer is not particularly limited; however, it can be confirmed by determination by the presence/absence of crystallization by visual observation, or XRD measurement of the vapor deposited film, and sharp diffraction peaks not being observed.

### <Regarding Cove Region>

An aspect of the present invention is in discovering that a compound having the cove region is promising as a photoelectric conversion element material for imaging elements. In the backbone represented by Formula (1), it is known that twisting occurs in the conjugated system to eliminate the steric repulsion between X¹ and X² by the existence of the cove region. Due to this effect, the photoelectric conversion element material for imaging elements which is the aspect of the present invention is superior in amorphous stability of the vapor deposited film compared to a material with a conventional planar polycyclic compound as the core. Additionally, with the photoelectric conversion element material for imaging elements which is an aspect of the present invention, the effect of stacking in a column shape can be expected with the nuclei overlapping each other, caused by twisting of the conjugated system. The photoelectric conversion element material for imaging elements, which is an aspect of the present invention, was assumed to be able to realize superior dark current for a photoelectric conversion element for imaging elements, by the amorphous film of the vapor deposited film is stable from the twisted polycyclic compound having a cove region forming the core, and the cores being arranged regularly to some extent even in the deposited film to secure a favorable change transport path. Furthermore, an aspect of the present invention can add characteristics as necessary such as adjustment of HOMO level, improvement in glass transition temperature, improvement in amorphous property, improvement in charge transport property, and improvement in blocking property against reverse charge, by this backbone being substituted. For example, in the case of using the photoelectric conversion element material for imaging elements which is an aspect of the present invention as a hole transport layer of a photoelectric conversion element for imaging elements, the effect of blocking electrons which are reverse charge simultaneously with favorably transporting holes can be expected. By blocking reverse charge, it is possible to suppress dark current in the photoelectric conversion element for imaging elements.

These structural characteristics are speculated to exhibit a function as a photoelectric conversion element material for imaging elements as explained below.

<Photoelectric Conversion Element for Imaging Elements>

A photoelectric conversion element for imaging elements according to an aspect of the present invention includes the photoelectric conversion element material for imaging elements according to the embodiment of the present invention. The configuration of the photoelectric conversion element for imaging elements is not particularly limited; however, for example, the configurations of (i) to (vi) shown below can be exemplified.

(i): lower electrode/photoelectric conversion layer/upper electrode
(ii): lower electrode/hole transport layer (electron blocking layer)/photoelectric conversion layer/upper electrode
(iii) lower electrode/photoelectric conversion layer/electron transport layer (hole blocking layer)/upper electrode
(iv): lower electrode/hole transport layer (electron blocking layer)/photoelectric conversion layer/electron transport layer (hole blocking layer)/upper electrode
(v): lower electrode/buffer layer/hole transport layer (electron blocking layer)/photoelectric conversion layer/electron transport layer (hole blocking layer)/upper electrode
(vi): lower electrode/hole transport layer/electrode blocking layer/photoelectric conversion layer/hole blocking layer/electron transport layer/upper electrode

It should be noted that a buffer layer may be replaced by a layer with another name or function, as necessary. As the layer having another name or function, for example, work function adjustment layer or the like can be exemplified.

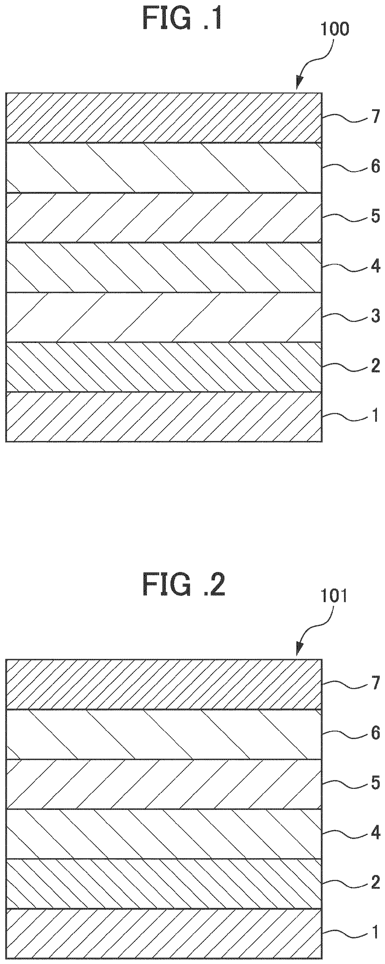

Hereinafter, the photoelectric conversion element for imaging elements according to an aspect of the present invention will be explained in detail while referencing Fig. 1, giving the configuration of the above (v) as an example. Fig. 1 is a schematic cross-sectional diagram showing an example of a laminate configuration of the photoelectric conversion element for imaging elements containing the photoelectric conversion element material for imaging elements according to an aspect of the present invention.

The photoelectric conversion element for imaging elements 100 includes, in this order, a base 1, lower electrode 2, buffer layer 3, hole transport layer 4, photoelectric conversion layer 5, electron transport layer 6 and upper electrode 7. Herein, the hole transport layer 4 and electron transport layer 6 may respectively be named electron blocking layer and hole blocking layer. However, part of the layer among these layers may be omitted, and yet another layer may be added.

The photoelectric conversion element for imaging elements shown in Fig. 1 has light incident from below the lower electrode 2, which is transparent. In addition, to the photoelectric conversion element for imaging elements, voltage is applied so that holes migrate to the lower electrode 2, and electrons migrate to the upper electrode 7, among the charge generated in the photoelectric conversion layer 5 (holes and electrons). In other words, the lower electrode 2 is defined as the hole collection electrode, and the upper electrode 7 is defined as the electron collection electrode.

### (Layer containing Photoelectric Conversion Element Material for Imaging Elements)

The photoelectric conversion element for imaging elements includes the photoelectric conversion element material for imaging elements in at least one layer selected from the group consisting of the photoelectric conversion layer and a layer between this photoelectric conversion layer and lower electrode. Therefore, the photoelectric conversion element for imaging elements 100 in the configuration example shown in Fig. 1 includes the photoelectric conversion element material for imaging elements in at least one layer selected from the group consisting of the photoelectric conversion layer 5 and hole transport layer 4. In particular, it is preferable for the hole transport layer 4 to contain the photoelectric conversion element material for imaging elements.

It should be noted that the photoelectric conversion element material for imaging elements may be contained in a plurality of layers possessed by the photoelectric conversion element for imaging elements, and in the case of an electron blocking layer being provided between the photoelectric conversion layer and hole transport layer, the electron blocking layer may include the photoelectric conversion element material for imaging elements.

Hereinafter, the photoelectric conversion element for imaging elements 100 in which the hole transport layer 4 contains the photoelectric conversion element material for imaging elements will be explained.

### (Substrate 1)

The substrate is not particularly limited, and for example, a glass plate, quartz plate, plastic plate or the like can be exemplified. In addition, in the case of a configuration in which light is incident from the side of the substrate 1, the substrate 1 is transparent to the wavelength of light.

### (Lower Electrode 2)

The lower electrode 2 is provided on the substrate 1. In the case of a photoelectric conversion element for imaging elements of a configuration in which light passes through the lower electrode and is incident on the photoelectric conversion layer, the lower electrode is formed with a transparent material that transmits or substantially transmits this light.

The transparent materials that can be used in the lower electrode 2 are not particularly limited; however, for example, indium tin oxide (ITO); indium zinc oxide (IZO), tin oxide, aluminum-doped tin oxide, magnesium-indium oxide, nickel-tungsten oxide, other metal oxides, metal nitrides such as gallium nitride, metal selenides such as zinc selenide, metal sulfides such as zinc sulfide and the like can be exemplified.

It should be noted that, in the case of a photoelectric conversion element for imaging elements of a configuration in which light only from the upper electrode side is incident on the photoelectric conversion layer, the transmission properties of the lower electrode is not important. Therefore, as an example of a material which can be used in the lower electrode in this case, iridium molybdenum palladium, platinum and the like can be exemplified.

### (Buffer Layer 3)

Between the lower electrode 2 and hole transport layer 4 described later, the buffer layer 3 is provided. The buffer layer 3 also has a role of efficiently receiving holes from the hole transport layer (electron blocking layer) 4, by adjusting the work function, and is also called a hole injection layer or work function adjusting layer. As a specific example of a conventionally known buffer layer 3, naphthalene-1,4,5,8-tetracarboxylic dianhydride (NTCDA), 2,3,6,7,10,11-hexacyano-1,4,5,8,9,12-hexaazatriphenylene (HATCN) and the like can be exemplified.

### (Hole Transport Layer (Electron Blocking Layer) 4)

Between the buffer layer 3 and photoelectric conversion layer 5, the hole transport layer (also called electron blocking layer) 4 is provided.

The hole transport layer 4 has a role of transporting holes produced by the photoelectric conversion layer 5 from the photoelectric conversion layer 5 to the side of the lower electrode 2, and a role of blocking electrons produced by the photoelectric conversion layer 5 from migrating to the side of the lower electrode 2. The hole transport layer 4 preferably contains the photoelectric conversion element for imaging elements as described above.

The hole transport layer 4 may be a single layer structure consisting of one type or two or more types of materials, or may be a laminate structure consisting of a plurality of layers of the same composition or different compositions. In other words, a material having both hole transport property and electron blocking property may be formed in a single-layer structure as the hole transport layer, and after forming the material specialized in the hole transport property is formed as the hole transport layer, a material specialized in electron blocking property may be formed on the hole transport layer as the electron blocking layer to make a laminate structure.

The hole transport layer 4 may further contain a conventionally known hole transport material in addition to the above photoelectric conversion element material for imaging elements. As the conventionally known hole transport material, aromatic tertiary amine compounds, naphthalene compounds, anthracene compounds, tetracene compounds, pentacene compounds, phenanthrene compounds, pyrene compounds, perylene compounds, fluorene compounds, carbazole compounds, indole compounds, pyrrole compounds, picene compounds, thiophene compounds, benzotrifuran compounds, benzotrithiophene compounds, naphthodithiophene compounds, naphthothienothiophene compounds, benzodifuran compounds, benzodithiophene compounds, benzothiophene compounds, naphthobisbenzothiophene compounds, chrysenodithiophene compounds, benzothienobenzothiophene compounds, indolocarbazole compounds and the like can be exemplified. Among these, fluorene compounds, naphthodithiophene compounds, naphthothienothiophene compounds, benzodifuran compounds, benzothiophene compounds, naphthobisbenzothiophene compounds, chrysenodithiophene compounds, benzothienobenzothiophene compounds, indolocarbazole compounds, etc. are preferred, and in particular fluorene compounds, chrysenodithiophene compounds, benzothienobenzothiophene compounds, and indolocarbazole compounds are preferable.

As specific examples of the conventionally known hole transport material, 9,9'-(9,9'-spirobi[9H-fluorene]-2,7'-diyl)bis[9H-carbazole], 2,7-diphenyl[1]benzothieno[3,2-b] [1]benzothiophene (DiPh-BTBT), benzo[1,2-b:3,4-b':5,6-b"]trifuran compound, benzo[1,2-b:3,4-b': 5,6-b"]trithiophene compound, naphtho[1,2-b:5,6-b']dithiophene, naphtho[2,3-b]naphtho[2',3':4,5]thieno [2,3-d]thiophene, benzo[1,2-b:4,5-b']difuran, benzo[1,2-b:4,5-b']dithiophene, benzo[1,2-b :4,5-b']bis[1]benzothiophene, naphtho[1,2-b:5,6-b']bis[1]benzothiophene, chryseno[1,2-b:8,7-b '] dithiophene, [1]benzothieno[3,2-b][1]benzothiophene, the following compounds (ic-1), (ic-2), and the like can be exemplified.

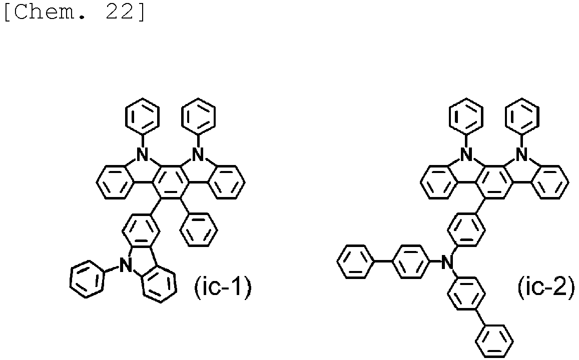

### (Photoelectric Conversion Layer 5)

Between the hole transport layer 4 and electron transport layer 6 described later, the photoelectric conversion layer 5 is provided. As the material of the photoelectric conversion layer 5, materials having a photoelectric conversion function can be exemplified.

The photoelectric conversion layer 5 may be a single layer structure consisting of one type or two or more types of materials, or may be a laminate structure consisting of a plurality of layers of the same composition or different compositions. As materials that can be used in the photoelectric conversion layer 5, which is a single-layer structure consisting of one type of material, for example, (1) coumarin and derivatives thereof, quinacridone and derivatives thereof, phthalocyanine and derivatives thereof, etc. can be exemplified. As materials that can be used in the photoelectric conversion layer 5, which is a single-layer structure consisting of two types of material, for example, a combination of the aforementioned (i) coumarin and derivatives thereof, quinacridone and derivatives thereof, phthalocyanine and derivatives thereof, and (ii) fullerene and derivatives thereof can be exemplified. Preparation of the photoelectric conversion layer 5 consisting of these materials may form by vapor depositing in a state mixing powders in advance, or may form by codepositing in any proportions. As the materials that can be used in the photoelectric conversion layer 5, which is a single-layer structure consisting of three types of materials, a combination of the aforementioned (i) coumarin and derivatives thereof, quinacridone and derivatives thereof, phthalocyanine and derivatives thereof, (ii) fullerene and derivatives thereof, and (iii) hole transport material can be exemplified. Preparation of the photoelectric conversion layer 5 consisting of these materials may form by vapor depositing in a state mixing powders in advance, or may form by codepositing in any proportion.

As specific examples of (i) coumarin derivatives, coumarin 6 and coumarin 30 can be exemplified. As specific examples of the quinacridone derivatives, N,N-dimethylquinacridone can be exemplified. As specific examples of the phthalocyanine derivative, boron subnaphthalocyanine chloride and boron subnaphthalocyanine chloride (SubNC) can be exemplified. As specific examples of the (ii) fullerene and derivatives thereof, [60] fullerene, [70] fullerene, and [6,6]-phenyl-C61-methyl butyrate ([60]PCBM) can be exemplified. As preferred compounds and specific examples of the (iii) hole transport material, the same as those described in the aforementioned hole transport layer 4 can be exemplified.

In addition, the material having a photoelectric conversion function is not limited to being contained only in the photoelectric conversion layer. For example, the material having a photoelectric conversion function may be contained in a layer (hole transport layer 4, or electron transport layer 6) adjacent to the photoelectric conversion layer 5.

### (Electron Transport Layer (Hole Blocking Layer) 6)

Between the photoelectric conversion layer 5 and upper electrode 7 described later, the electron transport layer (also called hole blocking layer) 6 is provided. The electron transport layer 6 has a role of transporting electrons produced by the photoelectric conversion layer 5 to a side of the upper electrode 7, and a role of blocking the holes produced by the photoelectric conversion layer 5 from migrating to the side of the upper electrode 7.

In addition, a conventionally known electron transport material can be contained in the electron transport layer 6. As the conventionally known electron transport material, for example, bis(8-hydroxyquinolinate)manganese, tris(8-hydroxyquinolinate)aluminum, tris(2-methyl-8-hydroxyquinolinate)aluminum, BCP (2,9-dimethyl-4,7-diphenyl-1,10-phenanthroline), Bphen (4,7-diphenyl-1,10-phenanthroline), BAlq (bis(2-methyl-8-quinolinolate)-4-(phenylphenolate) aluminum), 4,6-bis(3,5-di(pyridin-4-yl)phenyl)-2-methylpyrimidine, N,N'-diphenyl-1,4,5,8-naphthalenetetracarboxylic acid diimide, N,N'-di(4-pyridyl)-1,4,5,8-naphthalenetetracarboxylic acid diimide and the like can be exemplified.

The electron transport layer 6 may be a single layer structure consisting of one type or two or more types of materials, or may be a laminate structure consisting of a plurality of layers of the same composition or different compositions.

### (Upper Electrode 7)

The upper electrode 7 is provided on the electron transport layer 6. As the material of the upper electrode 7, sodium, sodium-potassium alloy, magnesium, lithium, magnesium/copper mixture, silver, magnesium/silver mixture, magnesium/aluminum mixture, magnesium/indium mixture, aluminum/aluminum oxide (Al₂O₃) mixture, indium, lithium/aluminum mixture, rare earths metal and the like can be exemplified.

### (Formation Method of Each Layer)

Each layer excluding the lower electrode 2 and upper electrode 7 explained above can be formed by thin film forming the material of each layer (along with materials such as a binder resin as necessary, and a solvent) by a known method such as a vacuum deposition method, spin coating method, casting method, LB (Langmuir-Blodgett method) or the like. There are no particular restrictions in the film thickness of each layer formed in this way, and can be appropriately selected according to the situation; however, normally it is in the range of 5 nm to 5 µm.

The lower electrode 2 and upper electrode 7 can be formed by thin film forming the electrode materials by a method such as vapor deposition or sputtering. The pattern may be formed via a mask of the desired shape upon vapor deposition or sputtering, and after forming a thin film by vapor deposition, sputtering or the like, a pattern of the desired shape may be formed by photolithography.

The film thickness of the lower electrode 2 and upper electrode 7 is preferably no more than 1 µm, and is more preferably at least 10 nm and no more than 200 nm.

The lower electrode 2 and upper electrode 7 may replace a material constituting each as necessary, or may use the same material (also called inverted structure). As specific examples of using the same material, for example, when the lower electrode 2 is ITO, a case of forming the upper electrode 7 as an ITO layer using a sputtering method or the like can be exemplified. In the case of such a structure, it becomes a photoelectric conversion element for imaging elements of a configuration in which light passes through the upper electrode 7 and is incident on the photoelectric conversion layer 5. In addition, in the case of replacing the materials constituting the lower electrode 2 and upper electrode 7, the layers may be laminated in the reverse order to the configuration of the above (v). For example, the configuration shown in the below (I) to (IV) can be exemplified.
(I): lower electrode/electron transport layer (hole blocking layer)/photoelectric conversion layer/upper electrode
(II): lower electrode/photoelectric conversion layer/hole transport layer (electron blocking layer)/upper electrode
(III): lower electrode/electron transport layer (hole blocking layer)/photoelectric conversion layer/hole transport layer (electron blocking layer)/upper electrode
(IV): lower electrode/electron transport layer (hole blocking layer)/photoelectric conversion layer/hole transport layer (electron blocking layer)/buffer layer/upper electrode

In the configuration of the above (IV), in the case of the lower electrode and the upper electrode being transparent materials and materials not having transparency, similarly to the configuration of (v), it becomes a photoelectric conversion element for imaging elements in which light passes through the lower electrode and is incident on the photoelectric conversion layer; however, the applied voltage is in the opposite direction to the configuration of the aforementioned (v) (also called reverse bias). In other words, voltage is applied so that the holes and electrodes produced in the photoelectric conversion layer transport to the upper electrode side and lower electrode side, respectively. In addition, in the above-mentioned configuration, the buffer layer has a role of lowering the damage to the organic film, upon forming the upper electrode by a sputtering method.

The imaging element equipped with the photoelectric conversion element according to an aspect of the present invention, for example, can be applied to the imaging element of digital camera or digital video camera, and the imaging element built into a mobile telephone or the like.

### EXAMPLES

Hereinafter, the present invention will be explained in further detail based on examples; however, the present invention is not to be interpreted as limited in any way to these examples.

### (Element Example A-1: Manufacture of Photoelectric Conversion Element for Imaging Elements made using Compound (2C-7))

As shown in Fig. 2, a photoelectric conversion element for imaging elements 101 having a laminate configuration consisting of the substrate 1, lower electrode 2, hole transport layer 4, photoelectric conversion layer 5, electron transport layer 6 and upper electrode 7 was prepared, and the dark current of this photoelectric conversion element was evaluated.

### (Preparation of Substrate 1 and Lower Electrode 2)

As the substrate equipped with the lower electrode on the surface thereof, a glass substrate with ITO transparent electrode on which a 2-mm wide indium-tin oxide (ITO) film (film thickness of 110 nm) was patterned in a stripe shape was prepared. Next, after cleaning this substrate with isopropyl alcohol, surface treatment was performed with ozone ultraviolet cleaning.

### (Preparation of Vacuum Deposition)

Vacuum deposition of each layer was performed by a vacuum deposition method on the substrate on which surface treatment was conducted after cleaning, and each layer was formed to be layered. First, the glass substrate was introduced into a vacuum deposition chamber, and reduced in pressure to 7.0 × 10⁻⁵ Pa. Then, in the following order, each was manufactured following the film forming conditions of each layer.

### (Manufacture of Hole Transport Layer 4)

The compound (2C-7) purified by sublimation was formed in a film of 30 nm at a rate of 0.15 nm/sec to manufacture the hole transport layer 4. It should be noted that, in the present configuration, the buffer layer between the lower electrode 2 and hole transport layer 4 have not been manufactured.

### (Manufacture of Photoelectric Conversion Layer 5)

N,N-dimethylquinacridone was formed in a thin film of 100 nm at a rate of 0.30 nm/sec to manufacture the photoelectric conversion layer 5.

### (Manufacture of Electron Transport Layer 6)

Tris(8-hydroxyquinolinate) aluminum was formed in a thin film of 30 nm at a rate of 0.15 nm/sec to manufacture the electron transport layer 6.

### (Manufacture of Upper Electrode 7)

Finally, a metal mask was arranged so as to go straight with the ITO stripe on the substrate to form a film of the upper electrode 7. The upper electrode formed a film of 100 nm of aluminum. The film formation rate of aluminum was 3 nm/sec.

From the above, the photoelectric conversion element for imaging elements 101 of 4 mm² surface area such as that shown in Fig. 2 was manufactured. It should be noted that the film thickness of each was measured by a probe-type film thickness measurement instrument (DEKTAK manufactured by Bruker).

Furthermore, this element was sealed inside of a nitrogen atmosphere glovebox with under 1 ppm oxygen and moisture concentration. Sealing was performed using bisphenol F-type epoxy resin (manufactured by Nagase & Co., Ltd.) between a sealant cap made from glass and a film formation substrate (element).

In the photoelectric conversion element for imaging elements manufactured in the above way, when applying a voltage of 2.5 V as an absolute value so that the holes are transported to the side of the lower electrode 2, and the electrons are transports to the side of the upper electrode 7, the current in the dark (dark current) was evaluated. Measurement of dark current was evaluated using a source measure unit 2636B manufactured by Keithley Instruments.

It should be noted that dark current is the relative value with the result of element comparative example A-1 as the reference value (1.0). The obtained measurement results are shown in Table 1.

### (Element Examples A-2, A-3, and Element Comparative Example A-1)

In Element Example A-1, in place of compound (2C-7), other than respectively using compound (2C-25), (3Ja-186), 2,7-diphenyl[1]benzothieno[3,2-b][1]benzothiophene (DiPh-BTBT) in order, the photoelectric conversion element for imaging elements was manufactured by the same method as Element Example A-1 and evaluated. The obtained measurement results are shown in Table 1.

**[Table 1]**

| | Compound | Dark current (A/cm²) |
|---|---|---|
| Element Example A-1 | 2C-7 | 2E-02 |
| Element Example A-2 | 2C-25 | 2E-03 |
| Element Example A-3 | 3Ja-186 | 3E-03 |
| Element Comparative Example A-1 | DiPh-BTBT | 1.0 |

From Table 1, the photoelectric conversion element material for imaging elements according to an aspect of the present invention can provide dark current on the order of two or three orders of magnitude lower than the comparative example compound.

### (Element Example B-1: Manufacture of photoelectric conversion element for imaging elements made using compound (2C-1))

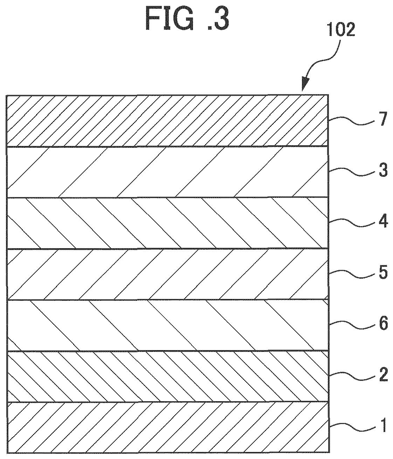

As shown in Fig. 3, the photoelectric conversion element for imaging elements 102 having the laminate configuration consisting of the substrate 1, lower electrode 2, electron transport layer 6, photoelectric conversion layer 5, hole transport layer 4, buffer layer 3 and upper electrode 7 was manufactured, and the dark current and external quantum efficiency of this photoelectric conversion element were evaluated.

### (Preparation of Substrate 1 and Lower Electrode 2)

For preparation of the substrate 1 and lower electrode 2 were conducted in the same procedure as Element Example A-1.

### (Manufacture of Electron Transport Layer 6)

The compound 4,6-bis(3,5-di(pyridin-4-yl)phenyl)-2-methylpyrimidine purified by sublimation was formed in a film of 10 nm at a rate of 0.10 nm/sec to manufacture the electron transport layer 6.

### (Manufacture of photoelectric conversion layer 5)

N,N-dimethylquinacridone and C60 were formed in a film of 120 nm in a ratio of 4:1 (mass ratio) to manufacture the photoelectric conversion layer 5. The film formation rate was 0.15 nm/sec.

### (Manufacture of Hole Transport Layer 4)

The compound (2C-1) purified by sublimation was formed in a film of 10 nm at a rate of 0.10 nm/sec to manufacture the hole transport layer 4.

### (Manufacture of Buffer Layer 3)

The compound 2,3,6,7,10,11-hexacyano-1,4,5,8,9,12-hexaazatriphenylene (HATCN) purified by sublimation was formed in a film of 10 nm at a rate of 0.10 nm/sec to manufacture the buffer layer 3.

### (Manufacture of Upper Electrode 7)

Finally, a metal mask was arranged so as to go straight with the ITO stripe on the substrate to form a film of the upper electrode 7. The upper electrode was formed in a film of 80 nm of silver. The film formation rate of silver was 0.1 nm/sec.

From the above, the photoelectric conversion element for imaging elements 102 of 4 mm² surface area such as that shown in Fig. 3 was manufactured. It should be noted that the film thickness of each was measured by a probe-type film thickness measurement instrument (DEKTAK manufactured by Bruker).

Furthermore, this element was sealed inside of a nitrogen atmosphere glovebox with under 1 ppm oxygen and moisture concentration. Sealing was performed using bisphenol F-type epoxy resin (manufactured by Nagase & Co., Ltd.) between a sealant cap made from glass and a film formation substrate (element).

When applying voltage of 2.5 V as an absolute value so that the electrons are transported to the opposite direction than Element Example A-1 in the photoelectric conversion element for imaging elements manufactured in the above way, i.e. to the side of the lower electrode 2, and the holes are transported to a side of the upper electrode 7, the current in the dark (dark current) and external quantum efficiency were evaluated. Measurement of dark current was evaluated using a source measure unit 2636B manufactured by Keithley Instruments. A solar cell spectral sensitivity measuring device (manufactured by Soma Opt.) was used in measurement of the external quantum efficiency. Measurement was performed with the wavelength of irradiated light of 560 nm, and intensity of 50 µW/cm². The results are shown in Table 2.

It should be noted that the dark current and external quantum efficiency are each relative values with the results of Element Comparative Example B-1 as reference value (1.0) and (100). The obtained measurement results are shown in Table 2.

### (Element Examples B-2 to B-19, and Element Comparative Example B-1)

In the manufacture of the hole transport layer (electron blocking layer) 4 of Element Example B-1, in place of compound (2C-1), other than respectively using compound (2C-2), compound (2C-3), compound (2C-7), compound (2C-8), compound (2C-12), compound (2C-13), compound (2C-26), compound (2C-32), compound (2C-61), compound (2C-107), compound (2C-108), compound (3Eb-194), compound (3Ia-91), compound (3Ia-194), compound ( 30a-88), compound (4Ac-192), compound (4Bb-192), compound (5A-0), or 2,7-diphenyl[1]benzothieno[3,2-b][1]benzothiophene (DiPh -BTBT), the photoelectric conversion element for imaging elements of Element Examples B-2 to B-19 and Element Comparative Example B-1 were manufactured by the same method as Element Example B-1, and the dark current and external quantum efficiency were measured by the same method as Element Example B-1. The results are shown in Table 2.

**[Table 2]**

| | Compound | Dark current (A/cm² ) | External quantum efficiency (%) |
|---|---|---|---|
| Element Example B-1 | 2C-1 | 3E-01 | 111 |
| Element Example B-2 | 2C-2 | 4E-02 | 108 |
| Element Example B-3 | 2C-3 | 9E-02 | 108 |
| Element Example B-4 | 2C-7 | 1E-01 | 104 |
| Element Example B-5 | 2C-8 | 1E-01 | 104 |
| Element Example B-6 | 2C-12 | 2E-01 | 105 |
| Element Example B-7 | 2C-13 | 1E-01 | 103 |
| Element Example B-8 | 2C-26 | 8E-03 | 102 |
| Element Example B-9 | 2C-32 | 7E-02 | 102 |
| Element Example B-10 | 2C-61 | 3E-01 | 107 |
| Element Example B-11 | 2C-107 | 5E-02 | 109 |
| Element Example B-12 | 2C-108 | 6E-02 | 107 |
| Element Example B-13 | 3Eb-194 | 5E-01 | 111 |
| Element Example B-14 | 3Ia-91 | 1.0 | 108 |
| Element Example B-15 | 3Ia-194 | 1.0 | 111 |
| Element Example B-16 | 30a-88 | 8E-02 | 107 |
| Element Example B-17 | 4Ac-192 | 4E-01 | 110 |
| Element Example B-18 | 4Bb-192 | 1.0 | 110 |
| Element Example B-19 | 5A-0 | 1E-01 | 110 |
| Element Comparative Example B-1 | DiPh-BTBT | 1.0 | 100 |

From Table 2, the photoelectric conversion element material for imaging elements according to an aspect of the present invention can provide higher external quantum efficiency than the comparative example compound.

Compound (2C-1), compound (2C-2), compound (2C-3), compound (2C-7), compound (2C-8), compound (2C-12), compound (2C-13), compound (2C-25), compound (2C-26), compound (2C-32), compound (2C-61), compound (2C-107), compound (2C-108), compound (3Eb-194), compound ( 3Ia-91), compound (3Ia-194), compound (3Ja-186), compound (30a-88), compound (4Ac-192), compound (4Bb-192), and compound (5a-0) used in the Examples and Comparative Examples were synthesized by the methods disclosed in Japanese Unexamined Patent Application, Publication No. 2019-34939, Japanese Unexamined Patent Application, Publication No. 2018-193371, Japanese Unexamined Patent Application, Publication No. 2020-15691, Japanese Unexamined Patent Application, Publication No. 2011-6397, Japanese Unexamined Patent Application, Publication No. 2019-34933, Japanese Unexamined Patent Application, Publication No. 2020-33332 and PCT International Publication No. WO2017/109722. Compound (DiPh-BTBT) used a sublimation grade product manufactured by Tokyo Chemical Industry Co., Ltd.

Although the present invention has been explained in detail by referencing specific embodiments, it will be evident to those skilled in the art that it is possible to apply various modifications and changes thereto without departing from the gist and scope of the present invention.

It should be noted that the entire contents of the specification, claims, drawings and abstract of Japanese Patent Application No. 2021-098353 filed on June 11, 2021, and Japanese Patent Application No. 2022-001855 filed on January 7, 2022 are cited herein and incorporated as a description of the disclosure of the present invention.

### EXPLANATION OF REFERENCE NUMERALS

1 substrate
2 lower electrode
3 buffer layer
4 hole transport layer (electron blocking layer)
5 photoelectric conversion layer
6 electron transport layer (hole blocking layer)
7 upper electrode
100 photoelectric conversion element for imaging elements
101 photoelectric conversion element for imaging elements
102 photoelectric conversion element for imaging elements

## Claims

1. A photoelectric conversion element material for imaging elements comprising a compound including a backbone having a cove region in a molecular structure, wherein the backbone may be substituted,
provided that an unsubstituted dibenzo[g,p] chrysene is not included as the compound.

2. The photoelectric conversion element material for imaging elements according to claim 1, wherein the backbone is represented by Formula (1) below, 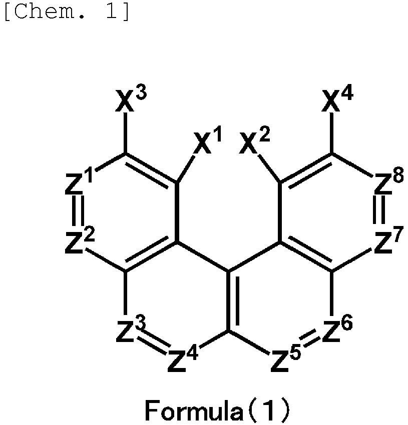 In Formula (1),
X¹ to X⁴ each independently represent a hydrogen atom or a substituent;
X¹ to X⁴ do not bond together to form a ring;
a plurality of X¹ to X⁴ may be the same or different;
Z¹ to Z⁸ each independently represent a nitrogen atom or a carbon atom that may have a substituent;
at least six of Z¹ to Z⁸ are carbon atoms that may have a substituent;
a plurality of Z¹ to Z⁸ may be the same or different; and
any of Z¹ to Z⁸, in a case of being a carbon atom having a substituent, may bond with a substituent on a carbon atom of any one or two of Z¹ to Z⁸ adjacent to the carbon atom to form a ring.

3. The photoelectric conversion element material for imaging elements according to claim 2, wherein at least seven of Z¹ to Z⁸ are carbon atoms that may have a substituent.

4. The photoelectric conversion element material for imaging elements according to claim 2, wherein Z¹ to Z⁸ are carbon atoms that may have a substituent.

5. The photoelectric conversion element material for imaging elements according to any one of claims 2 to 4, wherein X¹ and X² are hydrogen atoms.

6. The photoelectric conversion element material for imaging elements according to any one of claims 2 to 5, wherein Z³ and Z⁴ are carbon atoms that may have a substituent, and substituents of Z³ and Z⁴ bond together to form a ring.

7. A photoelectric conversion element material for imaging elements comprising a compound represented by any of Formulas (2A) to (5B) below, wherein 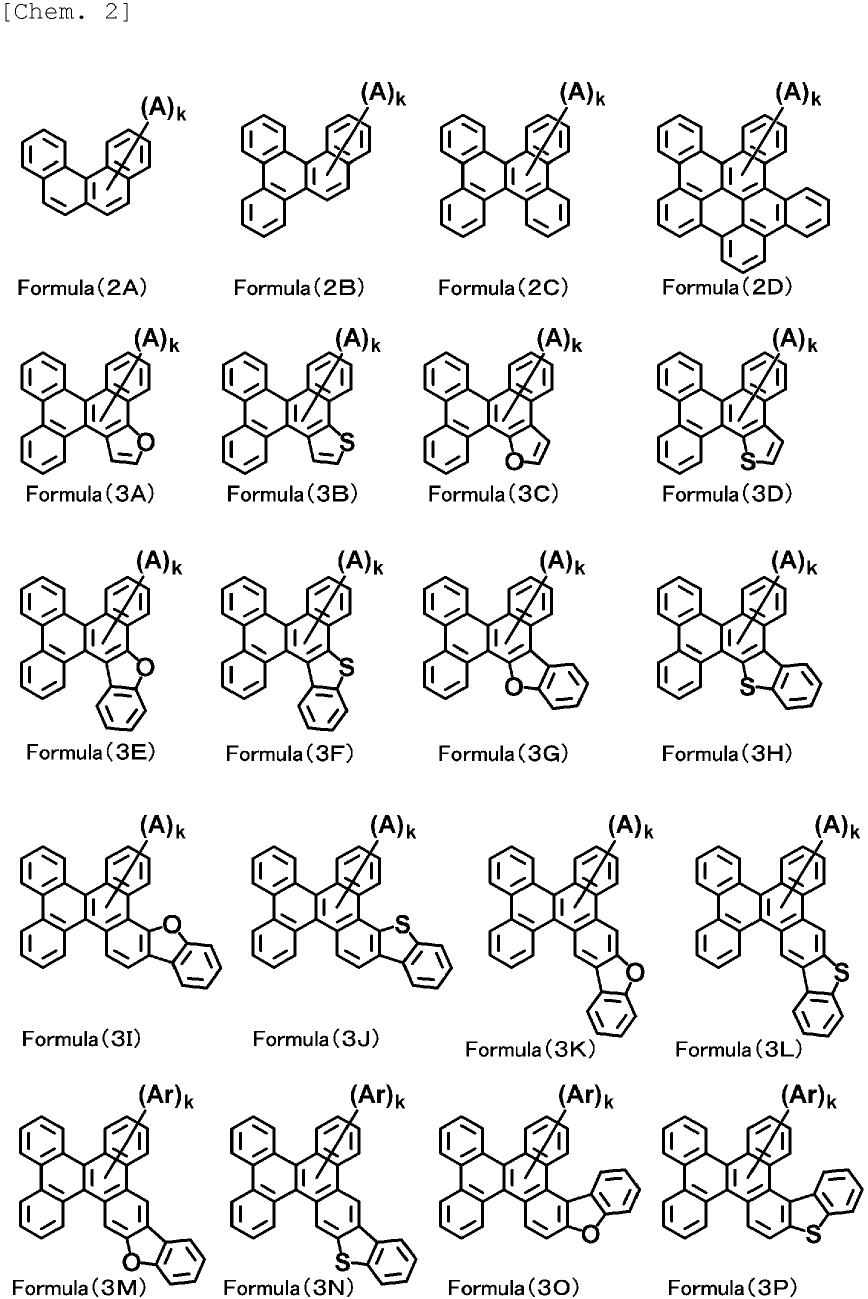 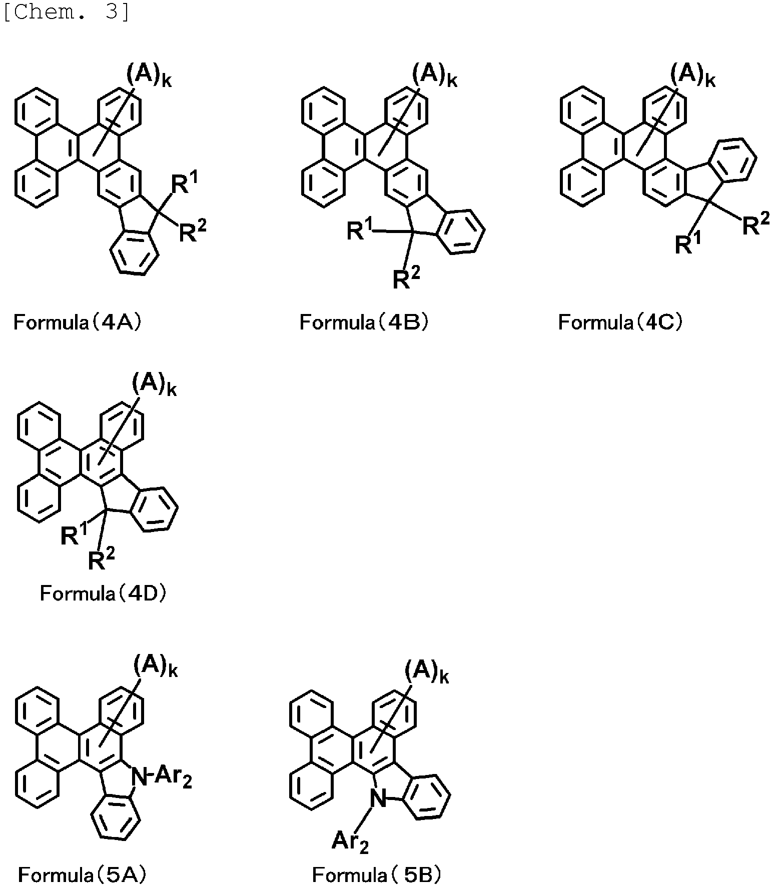
in Formulas (2A) to (5B),
A represents a charge transport substituent;
Ar₂ represents a monocyclic, linked or condensed aromatic hydrocarbon group of 6 to 30 carbon atoms that may have a substituent, a monocyclic, linked or condensed heteroaromatic group of 3 to 36 carbon atoms that may have a substituent, and a linear or branched alkyl group of 1 to 18 carbon atoms;
R¹ and R² each independently represent
a hydrogen atom;
a deuterium atom;
a monocyclic, linked or condensed aromatic hydrocarbon group of 6 to 30 carbon atoms that may have a substituent;
a monocyclic, linked or condensed heteroaromatic group of 3 to 36 carbon atoms that may have a substituent;
a linear or branched alkyl group of 1 to 18 carbon atoms; or
a linear or branched alkoxy group of 1 to 18 carbon atoms;
R¹ and R² may bond together to form a ring;
k represents an integer of 1 to 4; and
a plurality of A may be the same or different.

8. The photoelectric conversion element material for imaging elements according to claim 7, wherein the charge transport substituents are each independently
a deuterium atom;
a fluorine atom, chlorine atom, bromine atom or iodine atom;
a trifluoromethyl group or pentafluoroethyl group;
a cyano group;
a nitro group;
a hydroxy group;
a thiol group;
a monocyclic, linked or condensed aromatic hydrocarbon group of 6 to 30 carbon atoms that may have a substituent;
a monocyclic, linked or condensed heteroaromatic group of 3 to 36 carbon atoms that may have a substituent;
a phosphine oxide group that may have a substituent;
a silyl group that may have a substituent;
a boronyl group that may have a saturated hydrocarbon group of 2 to 10 carbon atoms;
a linear or branched alkyl group of 1 to 18 carbon atoms;
a linear or branched alkoxy group of 1 to 18 carbon atoms;
a trifluoromethylsulfonyloxy group; or
a group represented by Formula (6) or (6') below, wherein 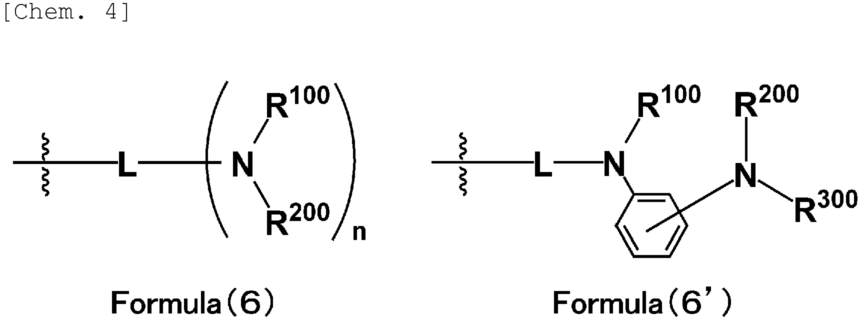
in the Formula,
R¹⁰⁰ to R³⁰⁰ each independently represent
a hydrogen atom or deuterium atom;
a monocyclic, linked or condensed aromatic hydrocarbon group of 6 to 30 carbon atoms that may have a substituent;
a monocyclic, linked or condensed heteroaromatic group of 3 to 36 carbon atoms that may have a substituent; or
a linear or branched alkyl group of 1 to 18 carbon atoms;
L each independently represent
a phenylene group that may be substituted by a methyl group or phenyl group,
a naphthylene group that may be substituted by a methyl group or phenyl group,
a biphenylene group that may be substituted by a methyl group or phenyl group, or
a single bond;
n represents 1 or 2;
when L is a single bond, n is 1;
when L is not a single bond, n is 1 or 2; and
when n is 2, a plurality of R¹⁰⁰ or R²⁰⁰ may be the same or different.

9. The photoelectric conversion element material for imaging elements according to any one of claims 1 to 8, wherein a molecular weight is no more than 1500.

10. The photoelectric conversion element material for imaging elements according to any one of claims 1 to 9, wherein a molecular weight is no more than 1000.

11. The photoelectric conversion element material for imaging elements according to any one of claims 1 to 10, wherein a HOMO value is 5.0 to 6.5 eV.

12. The photoelectric conversion element material for imaging elements according to any one of claims 1 to 11, wherein a band gap is 2.5 to 4.0 eV.

13. The photoelectric conversion element material for imaging elements according to any one of claims 1 to 12, wherein a LUMO value is 2.0 to 3.5 eV.

14. The photoelectric conversion element material for imaging elements according to any one of claims 1 to 13, wherein a glass transition temperature is 100°C or higher.

15. The photoelectric conversion element material for imaging elements according to any one of claims 1 to 14, wherein a glass transition temperature is 110°C or higher.

16. The photoelectric conversion element material for imaging elements according to any one of claims 1 to 15, wherein a glass transition temperature is 130°C or higher.

17. The photoelectric conversion element material for imaging elements according to any one of claims 1 to 16, wherein the photoelectric conversion element material for imaging elements is a photoelectric conversion element transport material for imaging elements, or a photoelectric conversion element charge blocking material for imaging elements.

18. The photoelectric conversion element material for imaging elements according to any one of claims 1 to 17, wherein the photoelectric conversion element material for imaging elements is a photoelectric conversion element hole transport material for imaging elements or a photoelectric conversion element electron blocking material for imaging elements.

19. A photoelectric conversion element for imaging elements, comprising the photoelectric conversion element material for imaging elements according to any one of claims 1 to 18.

20. The photoelectric conversion element for imaging elements according to claim 19, wherein a vapor deposited film of the photoelectric conversion element material for imaging elements is an amorphous layer.
